# EUROPEAN PATENT SPECIFICATION

(11) **EP 2 376 445 B1**
(45) Date of publication and mention of the grant of the patent: **29.05.2013**
(21) Application number: 09801825.2
(22) Date of filing: 24.12.2009
(51) Int. Cl.: C07C 323/65, C07C 323/60, C07C 317/44, A01N 37/34, A01N 37/36, A01N 41/10

(54) **SULFURE-CONTAINING COMPOUND AND USE THEREOF FOR CONTROLLING ARTHROPOD PEST**
SCHWEFELHALTIGE VERBINDUNG UND DEREN VERWENDUNG ZUR BEKÄMPFUNG VON ARTHROPODA-SCHÄDLINGEN
COMPOSÉ CONTENANT DU SOUFRE ET SON UTILISATION POUR LE CONTROLE DES NUISIBLES DE TYPE ARTHROPODE

(30) Priority: 24.12.2008 JP 2008327877
(43) Date of publication of application: 19.10.2011
(73) Proprietor: Sumitomo Chemical Company, Limited, Tokyo 104-8260 (JP)
(72) Inventor: MITSUDERA, Hiromasa, Westmount, QC (CA)
(74) Representative: Vossius & Partner
(86) International application number: PCT/JP2009/071873
(87) International publication number: WO 2010/074332

(56) References cited:
- WO-A1-2007/060839
- WO-A1-2008/143333

## Description

### Technical Field

The present invention relates to a novel sulfur-containing compound and use thereof.

### Background Art

Hitherto, various pesticidal compositions for controlling arthropod pests have been developed and put into practical use. For example, JP-Ar-2007-186494, JP-A 2007-161617 and JP-A 2007-055964 disclose halogen-containing organosulfur compounds, like WO 2008/143333, WO 2007/060839 and WO 2009/005110.

### Summary of Invention

### Technical Problem

An object of the present invention is to provide a novel compound having excellent control effect on arthropod pests, and the use of the compound for control of arthropod pests.

### Solution to Problem

The present inventors have intensively studied to find out a compound having an excellent controlling effect on arthropod pests. As a result, they have found that a sulfur-containing compound represented by the following formula (I) has an excellent controlling activity against arthropod pests such as insects and mites, and thus the present invention has been completed.

The present invention provides:
[1] A sulfur-containing compound represented by the formula (I): 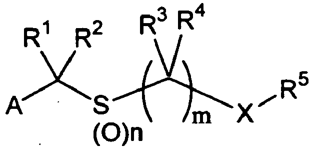 wherein m represents 1, 2 or 3, n represents 0, 1 or 2,
   A represents a C2-C10 fluoroalkyl group optionally substituted with a group selected from the group E,
   R¹ represents an optionally halogenated C1-C4 chain hydrocarbon group, a halogen atom, or a hydrogen atom,
   R² represents an optionally halogenated C1-C4 chain hydrocarbon group, -C(=G¹)R⁶, a cyano group, or a halogen atom,
   R³ and R⁴ independently represent an optionally halogenated C1-C4 chain hydrocarbon group, an optionally halogenated phenyl group, a halogen atom, or a hydrogen atom, and when m represents 2 or 3, two or more R³'s may be the same or different from each other and two or more R⁴'s may be the same or different from each other,
   X represents an oxygen atom, a sulfur atom, -SO-, or -SO₂-,
   R⁵ represents an optionally halogenated C1-C4 chain hydrocarbon group, -C(=G²)R⁷, a cyano group, or a hydrogen atom,
   G¹ and G² independently represent an oxygen atom or a sulfur atom,
   R⁶ represents an optionally halogenated C1-C4 alkyl group, a hydroxyl group, an optionally halogenated C1-C4 alkoxy group, an optionally halogenated C3-C6 alkenyloxy group, an optionally halogenated C3-C6 alkynyloxy group, an amino group, an optionally halogenated C1-C4 alkylamino group, an optionally halogenated di(C1-C4 alkyl)amino group, a C2-C5 cyclic amino group, or a hydrogen atom,
   R⁷ represents an optionally halogenated C1-C4 chain hydrocarbon group,
   The group E consists of -OR⁸, -SR⁸, -SO-R⁸, -SO₂-R⁸, a cyano group, a hydroxyl group, a chlorine atom, and a bromine atom, and
   R⁸ represents an optionally halogenated C1-C4 chain hydrocarbon group;
[2] The sulfur-containing compound according to [1], wherein R² is -C(=G¹)R⁶ or a cyano group;
[3] The sulfur-containing compound according to [1] or [2], wherein m is 2;
[4] The sulfur-containing compound according to [1] or [2], wherein m is 2, and R³ and R⁴ are hydrogen atoms;
[5] An arthropod pest-controlling composition comprising the sulfur-containing compound according to any one of [1] to [4] as an active ingredient; and
[6] A method for controlling an arthropod pest, which comprises applying an effective amount of the sulfur-containing compound according to any one of [1] to [4] to the arthropod pest or a habitat of the arthropod pest.

### Effects of Invention

The compound of the present invention has an excellent controlling effect on arthropod pests.

### Mode for Carrying Out the Invention

As used herein, for example, the "fluoroalkyl group" means an alkyl group substituted with one or more fluorine atoms. The expression "C1-C6" or the like, as used herein, means the total number of carbon atoms constituting-each substituent group.

As used herein, the "halogen atom" means a fluorine atom, a chlorine atom, a bromine atom, or an iodine atom.

The present invention includes each active isomer and an active mixture of the isomers at any ratio thereof, in cases where the present compound has stereoisomers originated from an asymmetric carbon atom which is connected with R¹ and R² or from an asymmetric carbon atom which is connected with R³ and R⁴, or in cases where the present compound has geometrical isomers originated from an alkenyl group.

Examples of the C2-C10 fluoroalkyl in the "C2-C10 fluoroalkyl group optionally substituted with a group selected from the group E" as used herein include a C2 fluoroalkyl group such as a 2-fluoroethyl group, a 2,2-difluoroethyl group, a 1,1,2,2-tetrafluoroethyl group, a 1,1,2,2,2-pentafluoroethyl group, and a 2,2,2-trifluoroethyl group;
a C3 fluoroalkyl group such as a 2-fluoropropyl group, 2,2-difluoropropyl group, a 3-fluoropropyl group, a 3,3-difluoropropyl group, a 3,3,3-trifluoropropyl group, a 2,2,3,3,3-pentafluoropropyl group, a 2,2,3,3-tetrafluoropropyl group, and a 1,1,2,2,3,3,3-heptafluoropropyl group;
a C4-fluoroalkyl group such as a 2-fluorobutyl group, a 2,2-difluorobutyl group, a 3-fluorobutyl group, a 3,3-difluorobutyl group, a 4-fluorobutyl group, a 4,4-difluorobutyl group, a 4,4,4-trifluorobutyl group, a 3,3,4,4,4-pentafluorobutyl group, a 2,2,3,4,4-pentafluorobutyl group, a 2,2,3,3,4,4,4-heptafluorobutyl group, a 1,1,2,2,3,3,4,4-octafluorobutyl group, a 1,1,2,2,3,3,4,4,4-nonafluorobutyl group, a 3,3,3-trifluoro-2-methylpropyl group, and a 2,3,3,3-tetrafluoro-2-(trifluoromethyl)propyl group;
a C5 fluoroalkyl group such as a 2-fluoropentyl group, a 2,2-difluoropentyl group, a 3-fluoropentyl group, a 3,3-difluoropentyl group, a 4-fluoropentyl group, a 4,4-difluoropentyl group, a 5-fluoropentyl group, a 5,5-difluoropentyl group, a 5,5,5-trifluoropentyl group, a 4,4,5,5,5-pentafluoropentyl group, a 3,3,4,4,5,5,5-heptafluoropentyl group, a 2,2,3,3,4,4,5,5-octafluoropentyl group, a 4,4,4-trifluoro-3-methylbutyl group, and a 2,2,3,3,4,4,5,5,5-nonafluoropentyl group;
a C6 fluoroalkyl group such as a 2-fluorohexyl group, a 2,2-difluorohexyl group, a 3-fluorohexyl group, a 3,3-difluorohexyl group, a 4-fluorohexyl group, a 4,4-difluorohexyl group, a 5-fluorohexyl group, a 5,5-difluorohexyl group, a 6-fluorohexyl group, a 6,6-difluorohexyl group, a 6,6,6-trifluorohexyl group, a 5,5,6,6,6-pentafluorohexyl group, a 4,4,5,5,6,6,6-heptafluorohexyl group, a 3,3,4,4,5,5,6,6,6-nonafluorohexyl group, a 3-(trifluoromethyl)pentyl group, and a 2,2,3,3,4,4,5,5,6,6,6-undecafluorohexyl group; a C7 fluoroalkyl group such as a 3-(trifluoromethyl)hexyl group and a 4-methyl-3-(trifluoromethyl)pentyl group; and a C8 fluoroalkyl group such as a 4,4-dimethyl-3-(trifluoromethyl)pentyl group.

The group E consists of -OR⁸ , -SR⁸, -SO-R⁸ , -SO²-R⁸, a cyano group, a hydroxyl group, a chlorine atom, and a bromine atom.

Examples of a group represented by "-OR⁸" include an optionally halogenated C1-C4 alkoxy group such as a 2-propynyloxy group and a 2-butynyloxy group; an optionally halogenated C2-C4 alkenyloxy group; an optionally halogenated C2-C4 alkynyloxy group; and an optionally halogenated C3-C6 cycloalkoxy group.

Examples of a group represented by "-SR⁸" include an optionally halogenated C1-C4 alkylthio group.

Examples of a group represented by "-SO-R⁸" include an optionally halogenated C1-C4 alkylsulfinyl group.

Examples of a group represented by "-SO₂-R⁸" include an optionally halogenated C1-C4 alkylsulfonyl group.

As used herein, examples of the "optionally halogenated C1-C4 chain hydrocarbon group" include an optionally halogenated C1-C4 alkyl group, an optionally halogenated C2-C4 alkenyl group, and an optionally halogenated C2-C4 alkynyl group.

Examples of the "an optionally halogenated C1-C4 alkyl group" as used herein include a C1-C4 alkyl group such as a methyl group, an ethyl group, a propyl group, a 1-methylethyl group (hereinafter, may be referred to as an i-propyl group), a 2,2-dimethylpropyl group, and a 1,1-dimethylethyl group (hereinafter may be referred to as a t-butyl group); and a C1-C4 haloalkyl group such as a chloromethyl group, a fluoromethyl group, a difluoromethyl group, a trifluoromethyl group, a 2,2,2-trifluoroethyl group, a 1,1,2,2-tetrafluoroethyl group, and a 1,1,2,2,2-pentafluoroethyl group.

Examples of the "optionally halogenated C2-C4 alkenyl group" as used herein include a C1-C4 alkenyl group such as a vinyl group, a 1-propenyl group, a 2-propenyl group, a 1-methyl-2-propenyl group, a 2-methyl-2-propenyl group, a 1-butenyl group, and a 2-butenyl group; and a C1-C4 haloalkenyl group such as a 2,2-difluorovinyl group, a 1,2,2-trifluorovinyl group, and a 3,3-difluoro-2-propenyl group.

Examples of the "optionally halogenated C2-C4 alkynyl group" as used herein include a C1-C4 alkynyl group such as an ethynyl group, a 1-propynyl group, a 2-propynyl group, a 1-methyl-2-propynyl group, a 1-butynyl group, a 2-butynyl group, and a 3-butynyl group; and a C1-C4 haloalkynyl group such as a 3,3,3-trifluoro-1-propynyl group.

As used herein, examples of the "optionally halogenated phenyl group" include a phenyl group; and a halogenated phenyl group such as a 4-chlorophenyl group, a 2-fluorophenyl group, and a 4-fluorophenyl group.

Examples of the "optionally halogenated C1-C4 alkoxy group" as used herein include a C1-C4 alkoxy group such as a methoxy group, an ethoxy group, and a propoxy group, and a C1-C4 haloalkoxy group such as a trifluoromethoxy group, a bromodifluoromethoxy group, a difluoromethoxy group, a chlorodifluoromethoxy group, a pentafluoroethoxy group, a 2,2,2-trifluoroethoxy group, and a 1,1,2,2-tetrafluoroethoxy group.

Examples of the "optionally halogenated C3-C6 alkenyloxy group" as used herein include a C3-C6 alkenyloxy group such as a 1=propynyloxy group, a 2-propenyloxy group, a 1-methyl-2-propenyloxy group, and a 1,1-dimethyl-2-propenyloxy group; and a C3-C6 haloalkenyloxy group such as a 2,2-difluoro-2-propenyloxy group.

Examples of the "optionally halogenated C3-C6 alkynyloxy group" as used herein include a C3-C6 alkynyloxy group such as a 2-propynyloxy group, a 1-methyl-2-propynyloxy group, a 1,1-dimethyl-2-propynyloxy group, a 2-butynyloxy group, a 1-methyl-2-butynyloxy group, and a 1,1-dimethyl-2-butynyloxy group; and a C3-C6 haloalkynyloxy group such as a 3,3,3-trifluoro-1-propynyloxy group.

Examples of the "optionally halogenated C1-C4 alkylamino group" as used herein include a C1-C4 alkylamino group such as an N-methylamino group, an N-ethylamino group, an N-propylamino group, and an N-(1-methylethyl)amino group; and a (C1-C4 haloalkyl)amino group such as an N-(2,2,2-trifluoroethyl)amino group.

Examples of the "optionally halogenated di(C1-C4 alkyl)amino group" as used herein include a di(C1-C4 alkyl)amino group such as an N,N-dimethylamino group, an N-ethyl-N-methylamino group, an N,N-diethylamino group, an N-methyl-N-propylamino group, an N-ethyl-N-propylamino group, an N,N-dipropylamino group, an N-methyl-N-(1-methylethyl)amino group, an N-ethyl-N-(1-methylethyl)amino group, and an N,N-di(1-methylethyl)amino group; and a di(C1-C4 haloalkyl)amino group such as an N-methyl-N-(2,2,2-trifluoroethyl)amino group and an N-methyl-N-ethyl-N-(2,2,2-trifluoroethyl)amino group.

Examples of the "C2-C5 cyclic amino group" as used herein include a 1-aziridino group, a 1-azetidinyl group, a 1-pyrrolidinyl group, a piperidino group, and a morpholino group.

Examples of the compound of the present invention include:
a compound of the formula (I), wherein m is 1;
a compound of the formula (I), wherein m is 2;
a compound of the formula (I), wherein m is 1, and R³ and
R⁴ are hydrogen atoms;
a compound of the formula (I), wherein m is 2, and R³ and R⁴ are hydrogen atoms;
a compound of the formula (I), wherein R¹ is a halogen atom or a hydrogen atom;
a compound of the formula (I), wherein R² i.s -C(=G¹)R⁶ (in which G¹ and R⁶ is as defined above) or a cyano group;
a compound of the formula (I), wherein R² is a cyano group;
a compound of the formula (I), wherein R¹ is a halogen atom or a hydrogen atom, and R² is -C(=G¹)R⁶ (in which G¹ and R⁶ are as defined above) or a cyano group;
a compound of the formula (I), wherein R¹ is a halogen atom or a hydrogen atom, and R² is -C(=G¹)R⁶ (in which G¹ and R⁶ are as defined above);
a compound of the formula (I), wherein R¹ is a halogen atom or a hydrogen atom, and R² is a cyano group;
a compound of the formula (I), wherein R¹ is an optionally substituted C1-C4 chain hydrocarbon group, and R² is - C(=G¹)R⁶, (in which G¹ and R⁶ are as defined above) or a cyano group;
a compound of the formula (I), wherein R¹ is an optionally substituted C1-C4 chain hydrocarbon group, and R² is a cyano group;
a compound of the formula (I), wherein R¹ is a methyl group, and R² is -C(=G¹)R⁶ (in which G¹ and R⁶ are as defined above) or a cyano group;
a compound of the formula (I), wherein R¹ is a methyl group and R² is a cyano group;
a compound of the formula (I), wherein R¹ is a chlorine atom, and R² is -C(=G¹)⁶ (in which G¹ and R⁶ are as defined above) or cyano group;
a compound of the formula (I), wherein R¹ is a chlorine atom and R² is a cyano group;
a compound of the formula (I), wherein R¹ is an optionally substituted C1-C4 chain hydrocarbon group, and R² is - C(=G¹)R⁶ (in which G¹ and R⁶ are as defined above) or a cyano group;
a compound of the formula (I), wherein R⁵ is an optionally substituted C1-C4 chain hydrocarbon group;
a compound of the formula (I), wherein X is an oxygen atom, and R⁵ is an optionally substituted C1-C4 chain hydrocarbon group;
a compound of the formula (I), wherein X is a sulfur atom, and R⁵ is an optionally substituted C1-C4 chain hydrocarbon group;
a compound of the formula (I), wherein A is a C2-C5 fluoroalkyl group, R¹ is a hydrogen atom or a C1-C4 alkyl group, R² is a cyano group, R³ and R⁴ independently represent a hydrogen atom or a C1-C4 alkyl group, R⁵ is a hydrogen atom or a C1-C4 alkyl group, and X is an oxygen atom or a sulfur atom; and
a compound of the formula (I), wherein A is a C2-C5 fluoroalkyl group, R¹ is a hydrogen atom, R² is a cyano group, R³ and R⁴ independently represent a hydrogen atom or a C1-C4 alkyl group, R⁵ is a hydrogen atom or a C1-C4 alkyl group, and X is an oxygen atom or a sulfur atom.

Then, a process for producing the compound of the present compound is explained.

Among the compounds of the present invention, a compound represented by the formula (I-a) wherein n is 0 can be produced, for example, by the following Production Process 1 to Production Process 5.

Hereinafter, a compound represneted by the formula (α) (α = arbitrary symbol) may be referred to as a "compound (α)".

### Production Process 1

The compound represented by the formula (I-a) can be produced, for example, by reacting a compound (a) and a compound (b) in the presence of a base: 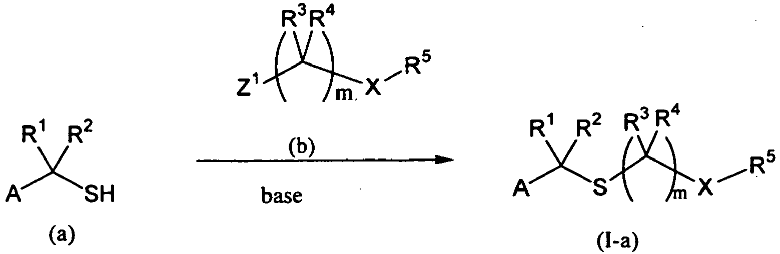 wherein A, X, R¹, R², R³, R⁴, R⁵ and m are as defined above, and Z¹ represents a leaving group such as a chlorine atom, a bromine atom, an iodine atom, a methanesulfonyl group or a p=toluenesulfonyl group.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydride, sodium hydroxide, potassium hydroxide, and potassium carbonate; alkali metal alkoxides such as sodium methoxide and potassium tert-butoxide; and organic bases such as triethylamine, 1,4-diazabicyclo[2.2.2]octane, and 1,8-diazabicyclo[5.4.0]-7-undecene.

The'amount of the base to be used is usually from 1 to 10 mol per 1 mol of the compound (a).

The amount of the compound (b) is usually from 1 to 10 mol per on 1 mol of the compound (a).

The reaction temperature is usually within a range from -50 to 100°C, and the reaction time is usually within a range from 1 to 24 hours.

After completion of the reaction, the compound (I-a) can be isolated, for example, by pouring the reaction mixture into water and extracting the mixture with an organic solvent, followed by concentration. The isolated compound (I-a) can be further purified by chromatography, recrystallization or the like.

### Production Process 2

The compound represented by the formula (I-a) can be also produced by reacting a compound (c) and a compound (d) in the presence of a base: 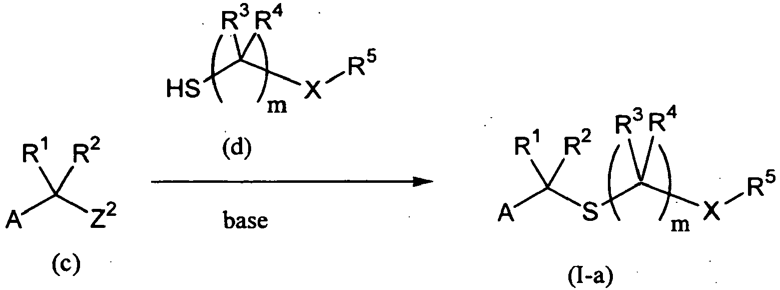 wherein A, X, R¹, R², R³, R⁴, R⁵ and m are as defined above, and Z² represents a leaving group such as a chlorine atom, a bromine atom, an iodine atom, a methanesulfonyl group, or a p-toluenesulfonyl group.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydride, sodium hydroxide, potassium hydroxide, and potassium carbonate; alkali metal alkoxides such as sodium methoxide and potassium tert-butoxide; and organic bases such as triethylamine, 1,4-diazabicyclo[2.2.2]octane, and 1,8-diazabicyclo[5.4.0]-7-undecene.

The amount of the base to be used is usually from 1 to 10 mol per on 1 mol of the compound (d).

The amount of the compound (c) is usually from 1 to 10 mol per 1 mol of the compound (d).

The reaction temperature is usually within a range from -50 to 100°C, and, the reaction time is usually within a range from 1 to 24 hours.

After completion of the reaction, the compound (1-a) can be isolated, for example, by pouring the reaction mixture into water and extracting the mixture with an organic solvent, followed by concentration. The isolated compound (I-a) can be further purified by chromatography, recrystallization or the like.

### Production Process 3

The compound represented by the formula (I-a) shown below can be also produced from the compound (c) by the following method: 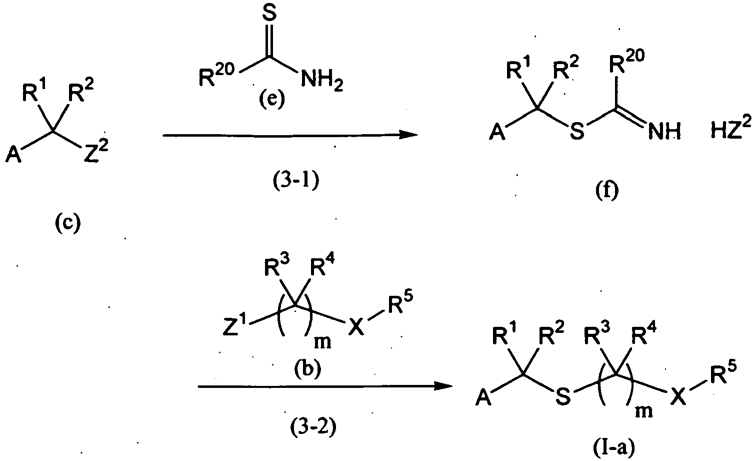 wherein A, X, R¹, R², R³, R⁴, R⁵, m, Z¹ and Z² are as defined above, and R²⁰ represents a methyl group or an amino group.

### Step (3-1)

The compound (f) can be produced by reacting the compound (c) with the compound (e).

The reaction is usually carried out in the presence of a solvent.

Examples of the solvent include halogenated hydrocarbons such as dichloromethane and chloroform; alcohols such as methanol and ethanol; and their mixtures.

The amount of the compound (e) is usually from 1 to 3 mol per 1 mol of the compound (c).

The reaction temperature is usually within a range from 20 to 200°C, and the reaction time is usually within a range from 0.5 to 240 hours.

After completion of the reaction, the compound (f) can be isolated, for example, by concentration of the reaction mixture. The isolated compound (f) can be used in the step (3-2) without purification, or can be further purified by recrystallization or the like, if necessary.

### Step (3-2)

The compound represented by the formula (I-a) can be prduced by reacting the compound (f) with the compound (b) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydroxide and potassium hydroxide; and alkali metal alkoxides such as sodium methoxide and potassium tert-butoxide.

The amount of the base to be used is usually from 1 to 50 mol per 1 mol of the compound (f).

The amount of the compound (b) usually from 1 to 10 mol per 1 mol of the compound (f).

The reaction can be carried out using a phase transfer catalyst such as tetra n-butylammonium bromide, if necessary. The amount of the phase transfer catalyst is usually from 0.05 to 1.0 mol per 1 mol of the compound (f).

The reaction temperature is usually within a range from -50 to 100°C, and the reaction time is usually within a range from 1 to 24 hours.

After completion of the reaction, the compound (I-a) can be isolated, for example, by pouring the reaction mixture into water and extracting the mixture with an organic solvent, followed by concentration. The isolated compound (I-a) can be further purified by chromatography, recrystallization or the like.

### Production Process 4

The compound represented by the formula (I-a) can be also produced from the compound (c) by the follwing method: 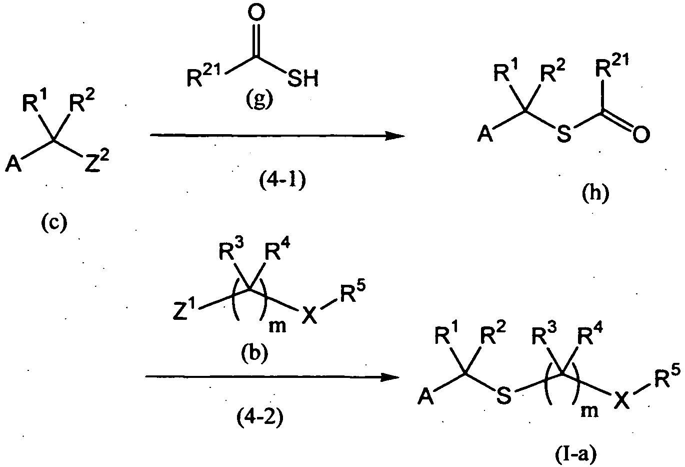 wherein A, X, R¹, R², R³, R⁴, R⁵, m, Z¹ and Z² are as defined above, and R²¹ represents a methyl group or a phenyl group.

### Step (4-1)

The compound (h) can be produced by reacting the compound (c) with the compound (g) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydride and potassium carbonate; and organic bases such as triethylamine, 1,4-diazabicyclo[2.2.2]octane, and 1,8-diazabicyclo[5.4.0]-7-undecene.

The amount of the base to be used is usually from 1 to 10 mol per 1 mol of the compound (c).

The amount of the compound (g) usually from 1 to 5 mol per 1 mol of the compound (c).

The reaction temperature is usually within a range from -20 to 80°C, and the reaction time is usually within a range from 1 to 24 hours.

After completion of the reaction, the compound (h) can be isolated, for example, by pouring the reaction mixture into acidic water (e.g., dilute hydrochloric acid) and extracting the mixture with an organic solvent, followed by concentration. The isolated compound (h) can be further purified by chromatography, recrystallization or the like.

### Step (4-2)

The compound represented by the formula (I-a) can be produced by reacting the compound (b) with the compound (h) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such a toluene and xylene; halogenated hydrocarbons such as 1,2-dichloroethane and chlorobenzene; water; and their mixtures.

Examples of the base include inorganic bases such as sodium hydroxide and potassium hydroxide; and alkali metal alkoxides such as sodium methoxide, sodium ethoxide and potassium tert-butoxide.

The amount of the base to be used is usually from 1 to 10 mol per 1 mol of the compound (h).

The amount of the compound (b) is usually from 1 to 10 mol per 1 mol of the compound (h).

The reaction temperature is within a range from -50 to 100°C, and the reaction time is usually within a range from 1 to 24 hours.

After completion of the reaction, the compound (I-a) can be isolated, for example, by pouring the reaction mixture into water and extracting the mixture with an organic solvent, followed by concentration. The isolated compound (I-a) can be further purified by chromatography, recrystallization or the like.

### Production Process 5

The compound represented by the formula (I-a) can be aslo produced from the compound (b) by the following method: 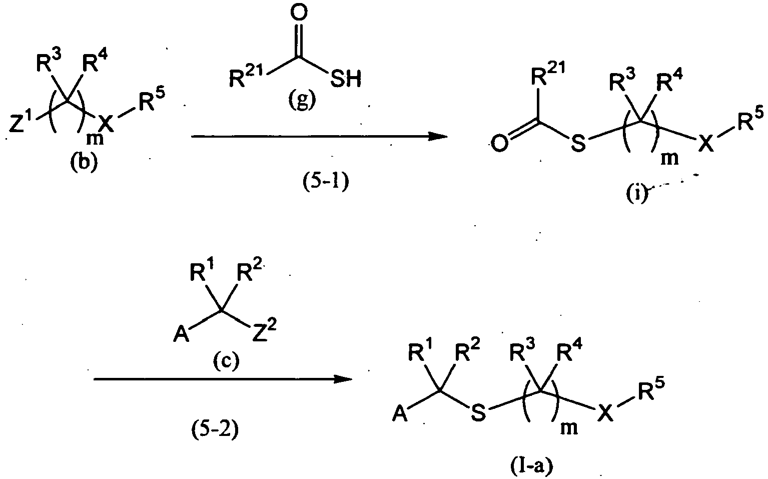 wherein A, X, R¹, R², R³, R⁴, R⁵, R²¹, m, Z¹ and Z² are as defined above.

### Step (5-1)

The compound (i) can be produced by reacting the compound (b) with the compound (g) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydride and potassium carbonate; and organic bases such as triethylamine, 1,4-diazabicyclo[2.2.2]octane, and 1,8-diazabicyclo[5.4.0]-7-undecene.

The amount of the base to be used is usually from 1 to 10 mol per 1 mol of the compound (b).

The amount of the compound (g) usually from 1 to 5 mol per 1 mol of the compound (b).

The reaction temperature is usually within a range from -20 to 80°C, and the reaction time is usually within a range from 1 to 24 hours.

After completion of the reaction, the compound (i) can be isolated, for example, by pouring the reaction mixture into acidic water (e.g., dilute hydrochloric acid) and extracting the mixture with an organic solvent, followed by concentration. The isolated compound (i) can be further purified by chromatography, recrystallization or the like.

### Step (5-2)

The compound represented by the formula (I-a) can be produced by reacting the compound (c) with the compound (i) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydroxide and potassium hydroxide; and alkali metal alkoxides such as sodium methoxide, sodium ethoxide and potassium tert-butoxide.

The amount of the base to be used is usually from 1 to 10 mol per 1 mol of the compound (i).

The amount of the compound (c) is usually from 1 to 10 mol per 1 mol of the compound (i).

The reaction temperature is within a range from -50 to 100°C, the reaction time is usually within a range from 1 to 24 hours.

After completion of the reaction, the compound (I-a) can be isolated, for example, by pouring the reaction mixture into water and extracting the mixture with an organic solvent, followed by concentration. The isolated compound (I-a) can be further purified by chromatography, recrystallization or the like.

### Production Process 6

Among the compounds of the present invention represented by the formula (I), a compound (I-b) in which R¹ is a hydrogen atom and R² is -C(=O)R⁶ or a cyano group can be produced by the step (6-1) shown below, and a compound (I-c) in which R¹ is an optionally halogenated C1-C4 chain hydrocarbon group and R² is -C(=O)R⁶ or a cyano group can be produced by the step (6-2) shown below: 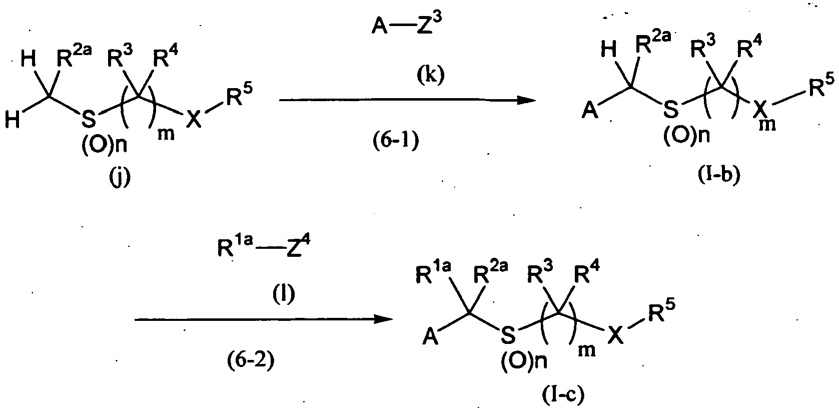 wherein A, X, R³, R⁴, R⁵, n and m are as defined above, Z³ and Z⁴ represent a leaving group such as a chlorine atom, a bromine atom, an iodine atom, a methanesulfonyl group, or a p-toluenesulfonyl group, R^{1a} represents an optionally halogenated C1-C4 chain hydrocarbon group, and R^{2a} repreesents -C(=O)R⁶ or a cyano group.

### Step (6-1)

The compound represented by the formula (I-b) can be produced by reacting the compound (k) with the compound (j) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and.heptane; aromatic hydrocarbons such as toluene and xylene; halogenated hydrocarbons such as 1,2-dichloroethane and chlorobenzene; water; and their mixtures.

Examples of the base include inorganic bases such as sodium hydride, sodium hydroxide, potassium hydroxide, and potassium carbonate; alkali metal alkoxides such as sodium methoxide and potassium tert-butoxide; and organic bases such as triethylamine, 1,4-diazabicyclo[2.2.2]octane, and 1,8-diazabicyclo[5.4.0]-7-undecene.

The amount of the base to be used is usually from 1 to 10 mol per 1 mol of the compound (j).

The compound (k) is usually from 1 to 10 mol per 1 mol of the compound (j).

The reaction temperature is usually within a range from -50 to 100°C, and the reaction time is usually within a range from 1 to 24 hours.

After completion of the reaction, the compound (I-b) can be isolated, for example, by pouring the reaction mixture into water and extracting the mixture with an organic solvent, followed by concentration. The isolated compound (I-b) can be further purified by chromatography, recrystallization or the like.

### Step (6-2)

The compound represented by the formula (I-c) can be produced by reacting the compound (1) with the compound (I-b) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydride, sodium hydroxide, potassium hydroxide, and potassium carbonate; alkali metal alkoxides such as sodium methoxide and potassium tert-butoxide; and organic bases such as triethylamine, 1,4-diazabicyclo[2.2.2]octane, and 1,8-diazabicyclo[5.4.0]-7-undecene.

The amount of the base to be used is usually from 1 to 10 mol per 1 mol of the compound (I-b).

The amount of the compound (1) is usually from 1 to 10 mol per 1 mol of the compound (I-b).

The reaction temperature is usually within a range from -50 to 100°C, and, the reaction time is usually within a range from 1 to 24 hours.

After completion of the reaction, the compound (I-c) can be isolated, for example, by pouring the reaction mixture into water and extracting the mixture with an organic solvent, followed by concentration. The isolated compound (I-c) can be further purified by chromatography, recrystallization or the like.

### Production Process 7

Among the compounds of the present invention represented by the formula (I), the compound (I-c) in which R¹ is an optionally halogenated C1-C4 chain hydrocarbon group and R² is -C(=O)R⁶ or a cyano group can also be produced from the compound (j) by the following method: 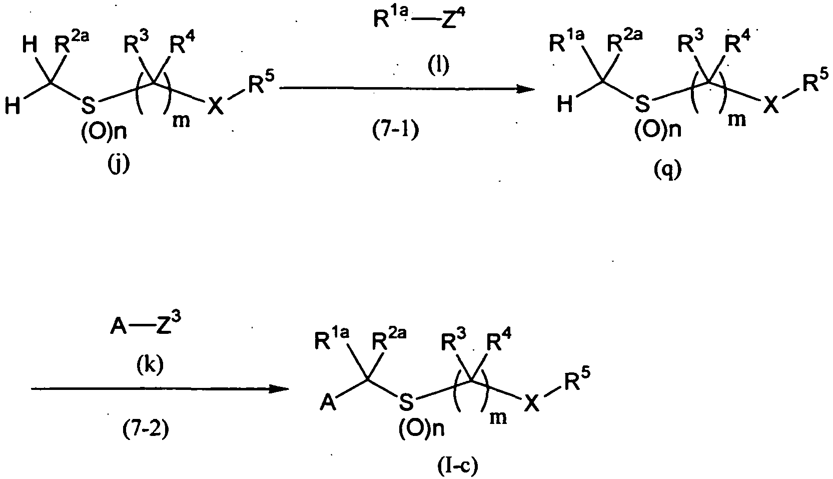 wherein A, X, R^{1a}, R^{2a}, R³, R⁴, R⁵, n, m, Z³ and Z⁴ are as defined above.

### Step (7-1)

The compound (q) can be produced by reacting the compound (l) with the compound (j) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydride, sodium hydroxide, potassium hydroxide, and potassium carbonate; alkali metal alkoxides such as sodium methoxide and potassium tert-butoxide; and organic bases such as triethylamine, 1,4-diazabicyclo[2.2.2]octane, and 1,8-diazabicyclo[5.4.0]-7-undecene.

The amount of the base to be used is usually from 1 to 10 mol per 1 mol of the compound (j).

The amount of the compound (l) is usually from 1 to 10 mol per 1 mol of the compound (j).

The reaction temperature is usually within a range from -50 to 100°C, and, the reaction time is usually within a range from 1 to 24 hours.

After completion of the reaction, the compound (q) can be isolated, for example, by pouring the reaction mixture into water and extracting the mixture with an organic solvent, followed by concentration. The isolated compound (q) can be further purified by chromatography, recrystallization or the like, if necessary.

### Step (7-2)

The compound represented by the formula (I-c) can be produced by reacting the compound (k) with the compound (q) in the presence of a base.

The reaction is usually carried out in the presence of a solvent.

Examples of the solvent include ethers such as diethyl ether, tetrahydrofuran, and dimethoxyethane; acid amides such as N,N-dimethylformamide; organosulphurs such as dimethyl sulfoxide and sulfolane; aliphatic hydrocarbons such as hexane and heptane; aromatic hydrocarbons such as toluene and xylene; halogenated hydrocarbons such as 1,2-dichloroethane and chlorobenzene; water; and their mixtures.

Examples of the base include inorganic bases such as sodium hydride, sodium hydroxide, potassium hydroxide, and potassium carbonate; alkali metal alkoxides such as sodium methoxide and potassium tert-butoxide; and organic bases such as triethylamine, 1,4-diazabicyclo[2.2.2]octane, and 1,8-diazabicyclo[5.4.0]-7-undecene.

The amount of the base to be used is usually from 1 to 10 mol per 1 mol of the compound (q).

The amount of the compound (k) is usually from 1 to 10 mol per 1 mol of the compound (q).

The reaction temperature is usually within a range from -50 to 100°C, and the reaction time is usually within a range from 1 to 24 hours.

After completion of the reaction, the compound (I-c) can be isolated, for example, by pouring the reaction mixture into water and extracting the mixture with an organic solvent, followed by concentration. The isolated compound (I-c) can be further purified by chromatography, recrystallization or the like, if necessary.

Reference Production Process 1

Among the compounds of the present invention represented by the formula (I), the compound in which R¹ is a halogen atom, and R² is -C(=O)R⁶ or a cyano group can be produced from the compound (I-b) by the following method. 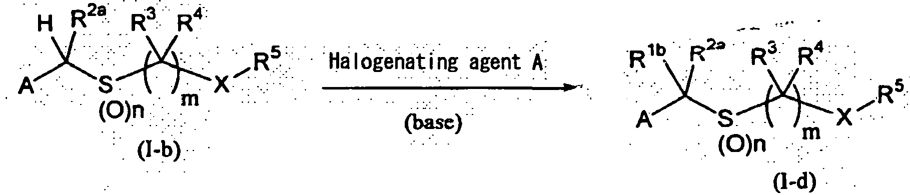 wherein A, X, R^{2a}, R³, R⁴, R⁵, n and m are as defined above, and R^{1b} represents a halogen atom

The reaction is usually carried out in the presence of a solvent.

Examples of the solvent include acid amides such as N,N-dimethylformamide; ethers such as diethyl ether and tetrahydrofuran; organosulphurs such as dimethyl sulfoxide and sulfolane; halogenated hydrocarbons such as chloroform, carbon tetrachloride, 1,2-dichloroethane, dichloromethane, and dichlorobenzene; aliphatic nitriles such as acetonitrile and propionitrile; aromatic hydrocarbons such as toluene and xylene; water; and their mixtures.

The reaction may be carried out in the presence of a base.

Examples of the base include inorganic bases such as sodium hydride, sodium hydroxide, potassium hydroxide, and potassium carbonate; alkali metal alkoxides such as sodium methoxide and potassium tert-butoxide; alkali metal amides such as lithium diisopropylamide; and organic bases such as triethylamine, 1,4-diazabicyclo[2.2.2]octane, and 1,8-diazabicyclo[5.4.0]-7-undecene.

The amount of the base to be used is usually from 1 to 10 mol per 1 mol of the compound (I-b).

Examples of the halogenating agent A include halogenated hydrocarbons such as carbon tetrachloride and hexachloroethane; halogens such as fluorine, chlorine, bromine, and iodine; N-halogenated succinimides such as N-chlorosuccinimide, N-bromosuccinimide, and N-iodosuccinimide; N-fluoropyridinium salts such as 1-fluoro-2,4,6-trimethylpyridinium trifluoromethanesulfonate and 1,1'-difluoro-2,2'-bipyridinium bistetrafluoroborate; and inorganic salts such as copper(II) chloride and copper(II) bromide.

The amount of the halogenating agent A is usually from 1 to 10 mol per 1 mol of the compound (I-b).

The reaction temperature is usually within a range from -100 to 100°C, and the reaction time is usually within a range from 1 to 24 hours.

After completion of the reaction, the compound (I-d) can be isolated, for example, by pouring the reaction mixture into water and extracting the mixture with an organic solvent, followed by concentration. The isolated compound (I-d) can be further purified by chromatography, recrystallization or the like, if necessary.

### Reference Production Process 2

Among the compound of the present invention represented by the formula (I), the compound in which R¹ is a halogen atom and R² is -C(=O)R⁵ or a cyano group can be produced from the compound (I-b) by the following method: 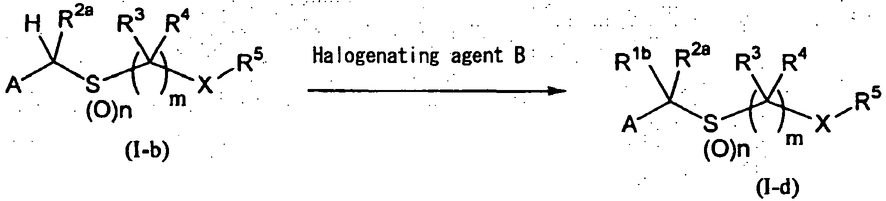 wherein A, X, R^{1b}, R^{2a}, R³, R⁴, R⁵, n and m are as defined above.

The reaction is usually carried out in the presence of a solvent.

Examples of the solvent include halogenated hydrocarbons such as chloroform, carbon tetrachloride, 1,2-dichloroethane, dichloromethane, and dichlorobenzene; aliphatic nitriles such as acetonitrile and propionitrile; aromatic hydrocarbons such as toluene and xylene; aliphatic carboxylic acids such as acetic acid; carbon disulfide; water; and their mixtures.

Examples of the halogenating agent B include halogens such as fluorine, chlorine, bromine, and iodine; hydrogen halides such as hydrogen fluoride, hydrogen chloride, hydrogen bromide, and hydrogen iodide; halogenated sulfur compounds such as thionyl chloride, thionyl bromide, and sulfuryl chloride; and halogenated phosphorus compounds such as phosphorus trichloride, phosphorus tribromide, phosphorus pentachloride, and phosphorus oxychloride.

The amount of the halogenating agent B is usually from 1 to 10 mol per 1 mol of the compound (I-b).

The reaction temperature is usually within a range from -100 to 200°C, and the reaction time is usually within a range from 1 to 24 hours.

After completion of the reaction, the compound (I-d) can be isolated, for example, by pouring the reaction mixture into water and extracting the mixture with an organic solvent, followed by concentration. The isolated compound (I-d) can be further purified by chromatography, recrystallization or the like, if necessary.

### Reference Production Process 3

Among the compounds of the present invention represented by the formula (I), a compound represented by the formula (I-e) in which n is 1 or 2 can be produced by oxidizing the compound represented by the formula (I-a) using an oxidizing agent: 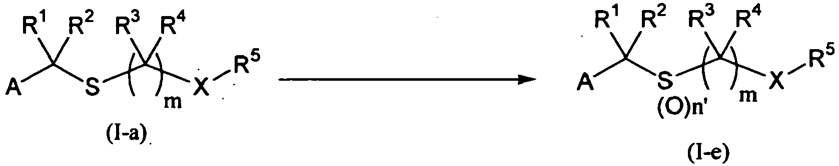 wherein A, X, R¹, R², R³, R⁴, R⁵ and m are as defined above, and n' represents 1 or 2.

The reaction is usually carried out in the presence of a solvent.

Examples of the solvent include alcohols such as methanol and ethanol; halogenated hydrocarbons such as dichloromethane and chloroform; aromatic hydrocarbons such as toluene and xylene; aliphatic carboxylic acids such as acetic acid and trifluoroacetic acid; water; and their mixtures.

Examples of the oxidizing agent include organic peroxides such as peracetic acid, trifluoroperacetic acid, and m-chloroperbenzoic acids; halogens such as chlorine and bromine; halogen-containing imides such as N-chlorosuccinimide; halides such as perchloric acid (or a salt thereof) and periodic acid (or a salt thereof); permaganates such as potassium permanganate; chromates such as potassium chromate; peroxysulfates such as potassium peroxysulfate; and hydrogen peroxide.

The amount of the the oxidizing agent to be used in the reaction is usually from 1 to 10 mol per 1 mol of the compound (I-a).

The reaction temperature is usually within a range from -50 to 200°C, and the reaction time is usually within a range from 1 to 72 hours.

After completion of the reaction, the compound (I-e) can be isolated, for example, by pouring the reaction mixture into water and extracting the mixture with an organic solvent, followed by concentration. The isolated compound (I-e) can be further purified by chromatography, recrystallization or the like, if necessary.

The compound (a), the compound (b), the compound (c), the compound (d), the compound (e) and the compound (g) can be prduced according to a known production method.

Examples of arthropod pests on which the compound of the present invention exhibits a controlling effect include harmful insects and harmful mites, and more specifically, the following arthropods.
Hemiptera:
   Planthoppers (Delphacidae) such as small brown planthopper (*Laodelphax striatellus*)*,* brown rice planthopper (*Nilaparvata lugens*)*,* and white-backed rice planthopper (*Sogatella furcifera*)*;* leafhoppers (Deltocephalidae) such as green rice leafhopper (*Nephotettix cincticeps*)*,* green rice leafhopper (*Nephotettix virescens*), and tea green leafhopper (*Empoasca onukii*)*;* aphids (Aphididae) such as cotton aphid (*Aphis gossypii*), green peach aphid (*Myzus persicae*)*,* cabbage aphid (*Brevicoryne brassicae*)*,* spiraea aphid (*Aphis spiraecola*)*,* potato aphid (*Macrosiphum euphorbiae*)*,* foxglove aphid (*Aulacorthum solani*)*,* oat bird-cherry aphid (*Rhopalosiphum padi*), tropical citrus aphid (*Toxoptera* citricidus), and mealy plum aphid (*Hyalopterus pruni*); stink bugs (Pentatomidae) such as green stink bug (*Nezara* antennata), bean bug (*Riptortus clavetus*), rice bug (*Leptocorisa chinensis*), white spotted spined bug (*Eysarcoris parvus),* and stink bug (*Halyomorpha mista*); whiteflies (Aleyrodidae) such as greenhouse whitefly (*Trialeurodes vaporariorum*), sweetpotato whitefly (Bemisia tabaci), citrus whitefly (*Dialeurodes citri*), and citrus spiny white fly (*Aleurocanthus spiniferus*); scales (Coccidae) such as Calfornia red scale (Aonidiella *aurantii*), San Jose scale (*Comstockaspis perniciosa*), citrus north scale (*Unaspis citri*), red wax scale (*Ceroplastes rubens*)., cottonycushion scale (*Icerya purchasi*), Japanese mealybug (*Planococcus* kraunhiae), Cosmstock mealybug (*Pseudococcus longispinus*), and white peach scale (*Pseudaulacaspis pentagona*); lace bugs (Tingidae); cimices such as *Cimex lectularius;* psyllids (Psyllidae), etc.;
Lepidoptera:
   Pyralid moths (Pyralidae) such as rice stem borer (*Chilo suppressalis*), yellow rice borer (*Tryporyza incertulas),* rice leafroller (*Cnaphalocrocis medinalis*), cotton leafroller (*Notarcha derogata*), Indian meal moth (*Plodia interpunctella*), *Ostrinia furnacalis,* cabbage webworm (*Hellula undalis*), and bluegrass webworm (*Pediasia teterrellus*); owlet moths (Noctuidae) such as common cutworm (*Spodoptera litura*), beet armyworm (*Spodoptera exigua*), armyworm (*Pseudaletia separata*), cabbage armyworm (*Mamestra brassicae*), black cutworm (*Agrotis ipsilon*), beet semi-looper (*Plusia nigrisigna*), *Thoricoplusia* spp., *Heliothis* spp., and *Helicoverpa* spp.; white butterflies (Pieridae) such as common white (*Pieris rapae*); tortricid moths (Tortricidae) such as *Adoxophyes* spp., oriental fruit moth (*Grapholita molesta*), soybean pod borer (*Leguminivora glycinivorella*), azuki bean podworm (*Matsumuraeses azukivora*), summer fruit tortrix (*Adoxophyes orana fasciata*), smaller tea tortrix (*Adoxophyes honmai*), oriental tea tortrix (*Homona magnanima*), apple tortrix (*Archips fuscocupreanus*), and codling moth (*Cydia pomonella*); leafblotch miners (Gracillariidae) such as tea leafroller (*Caloptilia theivora*), and apple leafminer (*Phyllonorycter ringoneella*); Carposinidae such as peach fruit moth (*Carposina niponensis*); lyonetiid moths (Lyonetiidae) such as *Lyonetia* spp.; tussock moths (Lymantriidae) such as *Lymantria* spp., and *Euproctis* spp.; yponomeutid moths (Yponomeutidae) such as diamondback (*Plutella xylostella*); gelechiid moths (Gelechiidae) such as pink bollworm (*Pectinophora gossypiella*), and potato tubeworm (*Phthorimaea operculella*); tiger moths and allies (Arctiidae) such as fall webworm (*Hyphantria cunea*); tineid moths (Tineidae) such as casemaking clothes moth (Tinea *translucens*), and webbing clothes moth (*Tineola bisselliella*), etc.;
Thysanoptera:
   Yellow citrus thrips (*Frankliniella occidentalis*), melon thrips (*Thrips palmi*), yellow tea thrips (*Scirtothrips dorsalis*), onion thrips (*Thrips tabaci*), flower thrips (*Frankliniella intonsa*), etc.;
Diptera:
   Culices (Calicidae) such as common mosquito (Culex *pipiens pallens*), *Culex tritaeniorhynchus,* and Southern house mosquito (*Culex quinquefasciatus*); Aedes spp. such as yellow fever mosquito (*Aedes aegypti*), and Asian tiger mosquito (Aedes *albopictus*); Anopheles spp. such as *Anopheles sinensis;* Chironomidae; Houseflies (Muscidae) such as housefly (*Musca domestica*), and false stable fly (*Muscina stabulans*); blow flies (Calliphoridae); flesh flies (Sarcophagidae); little house flies (Fanniidae); anthomyiid flies (Anthomyiidae) such as seedcorn maggot (*Delia platura*), and onion maggot (*Delia antiqua*); leafminer flies (Agromyzidae) such as rice leafminer (*Agromyza oryzae*), rice leafminer (*Hydrellia griseola*), tomato leafminer (*Liriomyza sativae*), legume leafminer (*Liriomyza trifolii*), and garden pea leafminer (*Chromatomyia horticola*); gout flies (Chloroidae) such as rice stem maggot (*Chlorops oryzae*); fruit flies (Tephritidae) such as melon fly (*Dacus cucurbitae*), and Meditteranean fruit fly (*Ceratitis capitata*); drosophila flies (Drosophilidae); humpbacked flies (Phoridae) such as *Megaselia spiracularis;* Psychodidae such as *Clogmia albipunctata;* Simuliidae; Tabanidae such as horsefly (*Tabanus trigonus*); stable flies (Stomoxys), etc.;
Coleoptera:
   Corn root worms (*Diabrotica* spp.) such as Western corn root worm (*Diabrotica virgifera virgifera*), and Southern corn root worm (Diabrotica undecimpunctata howardi); scarabs (Scarabaeidae) such as cupreous chafer (*Anomala cuprea*), soybean beetle (*Anomala rufocuprea*), and Japanese beetle (*Popillia japonica*); weevils (Curculionidae) such as maize weevil (*Sitophilus zeamais*), rice water weevil (*Lissorhoptrus oryzophilus*), azuki bean weevil (*Callosobruchus chinensis*), rice *curculio* (*Echinocnemus squameus*), boll weevil (*Anthonomus grandis*), and hunting billbug (*Sphenophorus venatus*); darkling beetles (Tenebrionidae) such as yellow mealworm (*Tenebrio molitor*), and red flour beetle (Tribolium castaneum); leaf beetles (Chrysomelidae) such as rice leaf beetle (*Oulema oryzae*), cucurbit leaf beetle (*Aulacophora femoralis*), striped flea beetle (Phyllotreta striolata), and Colorado beetle (*Leptinotarsa decemlineata*); dermestid beetles (Dermestidae) such as varied carper beetle (*Anthrenus verbasci*), and hide beetle (*Dermestes maculates*); deathwatch beetles (Anobiidae) such as cigarette beetle (*Lasioderma serricorne*); Epilachna such as twenty-eight-spotted ladybird (*Epilachna vigintioctopunctata*); bark beetles (Scolytidae) such as powder post beetle (*Lyctus brunneus*), and pine shoot beetle (*Tomicus piniperda*); false powderpost beetles (Bostrichidae); spider beetles (Ptinidae); longhorn beetles (Cerambycidae) such as white-spotted longicorn beetle (*Anoplophora malasiaca*); click beetles (*Agriotes* spp.); *Paederus fuscipes,* etc.;
Orthoptera:
   Asiatic locust (*Locusta migratoria*), African mole cricket (*Gryllotalpa africana*), rice grasshopper (Oxya *yezoensis*), rice grasshopper (*Oxya japonica*), Grylloidea, etc.;
Siphonaptera:
   Cat flea (*Ctenocephalides felis*), dog flea (*Ctenocephalides canis*), human flea (*Pulex* irritans), oriental rat flea (*Xenopsylla cheopis*), etc.;
Anoplura:
   Human body louse (*Pediculus humanus corporis*), crab louse (*Phthirus pubis*), short-nosed cattle louse (*Haematopinus eurysternus*), sheep louse (*Damalinia ovis*), hog louse (*Haematopinus suis*), etc.;
Hymenoptera:
   Ants (Formicidae) such as *Monomorium pharaonis, Formica* fusca *japonica,* black house ant (*Ochetellus glaber*), *Pristomyrmex pungens, Pheidole noda,* leaf-cutting ant (Acromyrmex spp.), and fire ant (*Solenopsis* spp.); hornets (Vespidae); bethylid wasps (Betylidae); sawflies (Tenthredinidae) such as Cabbage sawfly (Athalia *rosae*), and Athalia *japonica,* etc.;
Blattodea:
   Cockroaches (Blattariae) such as German cockroach (*Blattella germanica*), smokybrown cockroach (Periplaneta *fuliginosa),* American cockroach (*Periplaneta americana*), *Periplaneta brunnea,* and oriental cockroach (Blatta *orientalis*); and
   Termites (Termitidae) such as Japanese subterranean termite (*Reticulitermes speratus*), Formosan subterranean termite (*Coptotermes formosanus*), western drywood termite (*Incisitermes minor*), Daikoku drywood termite (Cryptotermes *domesticus*), *Odontotermes formosanus,* Neotermes *koshunensis, Glyptotermes satsumensis, Glyptotermes nakajimai, Glyptotermes fuscus, Glyptotermes kodamai, Glyptotermes kushimensis,* Japanese dampwood termite (*Hodotermopsis japonica*), *Coptotermes guangzhoensis, Reticulitermes miyatakei, Reticulitermes flavipes amamianus, Reticulitermes sp., Nasutitermes takasagoensis, Pericapritermes nitobei, Sinocapritermes mushae,* etc.;
Acarina:
   Spider mites (Tetranychidae) such as two-spotted spider mite (*Tetranychus urticae*), Kanzawa spider mite (*Tetranychus kanzawai*), citrus red mite (*Panonychus citri*), European red mite (*Panonychus ulmi*), and *Oligonychus* spp.; eriophyid mites (Eriophyidae) such as pink citrus rust mite (*Aculops pelekassi*), *Phyllocoptruta* citri, tomato rust mite (*Aculops lycopersici*), purple tea mite (*Calacarus carinatus*), pink tea rust mite (*Acaphylla theavagran*), *Eriophyes chibaensis,* and apple rust mite (Aculus *schlechtendali*); tarosonemid mites (Tarsonemidae) such as broad mite (*Polyphagotarsonemus latus*); false spider mites (Tenuipalpidae) such as *Brevipalpus phoenicis;* Tuckerellidae; ticks (Ixodidae) such as *Haemaphysalis longicornis, Haemaphysalis flava, Dermacentor taiwanicus,* American dog tick (*Dermacentor variabilis*), *Ixodes ovatus, Ixodes persulcatus,* black legged tick (*Ixodes scapularis*), lone star tick (Amblyomma americanum), *Boophilus microplus,* and *Rhipicephalus sanguineus;* Psoroptidae such as ear mite (Otodectes *cynotis*); itch mites (Sarcoptidae) such as *Sarcoptes scabiei;* folicle mites (Demodicidae) such as dog folicle mite (Demodex *canis*); acarid mites (Acaridae) such as mold mite (*Tyrophagus putrescentiae*), and *Tyrophagus similis;* house dust mites (Pyroglyphidae) such as *Dermatophagoides farinae,* and *Dermatophagoides ptrenyssnus;* cheyletide mites (Cheyletidae) such as *Cheyletus eruditus, Cheyletus malaccensis,* and *Cheyletus moorei*; parasitoid mites (Dermanyssidae) such as tropical rat mite (*Ornithonyssus bacoti*), northern fowl mite (*Ornithonyssus sylviarum*), and poultry red mite (*Dermanyssus gallinae*); chiggers (Trombiculidae) such as *Leptotrombidium akamushi;* spiders (Araneae) such as Japanese foliage spider (*Chiracanthium japonicum*), redback spider (Latrodectus *hasseltii*), etc.;
   Chilopoda: *Thereuonema hilgendorfi, Scolopendra subspinipes,* etc.;
   Diplopoda: garden millipede (*Oxidus gracilis*), *Nedyopus* tambanus, etc.;
   Isopoda: common pill bug (*Armadillidium vulgare*), etc.

Although the arthropod pest-controlling composition of the present invention may be the compound of the present invention itself, the arthropod pest-controlling composition of the present invention is usually in the form of a formulation such as an emulsifiable concentrate, an oil solution, a shampoo formulation, a flowable formulation, a dust, a wettable powder, a granule, a paste formulation, a microcapsule formulation, a foam formulation, an aerosol formulation, a carbon dioxide gas formulation, a tablet, or a resin formulation. The arthropod pest-controlling composition of the present invention may be processed into a poison bait, a mosquito coil, an electric mosquito mat, a smoking pesticide, a fumigant or a sheet, and then be used.

The arthropod pest-controlling composition of the present invention usually contains 0.1 to 95% by weight of the compound of the present invention.

The formulation of the arthropod pest-controlling composition of the present invention can be usually produced by mixing the compound of the present invention with a solid carrier, a liquid carrier and/or a gaseous carrier, and if necessary, with a surfactant or other pharmaceutical additives.

Examples of the solid carrier include finely-divided powder and granules of clay (e.g., kaolin clay, diatomaceous earth, bentonite,' agalmatolite clay (Fubasami clay), or acid clay), synthetic hydrated silicon oxide, talc, ceramics, other inorganic minerals (e.g., sericite, quartz, sulfur, activated carbon, calcium carbonate, or hydrated silica), and chemical fertilizers (e.g., ammonium sulfate, ammonium phosphate, ammonium nitrate, ammonium chloride, or urea).

Examples of the liquid carrier include aromatic or aliphatic hydrocarbons (e.g., xylene, toluene, alkylnaphthalene, phenylxylylethane, kerosene, light oil, hexane, or cyclohexane), halogenated hydrocarbons (e.g., chlorobenzene, dichloromethane, dichloroethane, or trichloroethane), alcohols (e.g., methanol, ethanol, isopropyl alcohol, butanol, hexanol, or ethylene glycol), ethers (e.g., diethylether, ethylene glycol dimethyl ether, diethylene glycol monomethyl ether, diethylene glycol monoethyl ether, propylene glycol monomethyl ether, tetrahydrofuran, or dioxane), esters (e.g., ethyl acetate, or butyl acetate), ketones (e.g., acetone, methyl ethyl ketone, methyl isobutyl ketone, or cyclohexanone), nitriles (e.g., acetonitrile, or isobutyronitrile), sulfoxides (e.g., dimethyl sulfoxide), acid amides (e.g., N,N-dimethylformamide, or N,N-dimethylacetamide), pyrrolidones (e.g., N,N-methyl-2-pyrrolidone, or N-octyl-2-pyrrolidone), propylene carbonate, ethyl lactate, 1,3-dimethyl-2-imidazolidinone, vegetable oils (e.g., soybean oil, or cottonseed oil), and vegetable essential oils (e.g., orange oil, hyssop oil, or lemon oil), water.

Examples of the gaseous carrier include butane gas, chlorofluorocarbon, liquefied petroleum gas (LPG), dimethyl ether, and carbon dioxide gas.

Examples of the surfactant include alkyl sulfate salts, alkyl sulfonate salts, alkylaryl sulfonate salts, alkyl aryl ethers and their polyoxyethylated derivatives, polyethylene glycol ethers, polyhydric alcohol esters, and sugar alcohol derivatives.

Examples of other pharmaceutical additives include a binder, a dispersant, and a stabilizer. Specific examples thereof include casein, gelatin, polysaccharides (e.g., starch, gum arabic, cellulose derivatives, or alginic acid), lignin derivatives, bentonite, saccharides, synthetic water-soluble polymers (e.g., polyvinyl alcohol, polyvinylpyrrolidone, or polyacrylic acid), PAP (isopropyl acid phosphate), BHT (2,6-di-t-butyl-4-methylphenol), BHA (a mixture of 2-t-butyl-4-methoxyphenol and 3-t-butyl-4-methoxyphenol), vegetable oils, mineral oils, fatty acids, and fatty acid esters.

Examples of a base material for a resin formulation include vinyl chloride polymers, and polyurethane. To the base material, if necessary, a plasticizer such as phthalate (e.g., dimethyl phthalate, or dioctyl phthalate), adipate, or stearic acid may be added. The resin formulation is obtained by kneading the compound of the present invention into the base material using a conventional kneading apparatus, followed by molding such as injection molding, extrusion molding, or press molding. The resulting resin formulation may be formed into the shape of a plate, a film, a tape, a net, a string or the like via a further step of molding, cutting, or the like, if necessary. These resin formulations may be used, for example, in the form of an animal collar, an animal ear tag, a sheet formulation, a lead, or a horticultural post.

Examples of a base material of a poison bait include cereal powder, vegetable oil, sugar, and crystalline cellulose. To the base material, if necessary, an antioxidant such as dibutylhydroxytoluene or nordihydroguaiaretic acid, a preservative such as dehydroacetic acid, an agent for preventing children or pets from eating the poison bait by mistake such as hot pepper powder, a pest-attractive perfume such as cheese perfume, onion perfume or peanut oil or the like may be added.

The arthropod pest-controlling composition of the present invention can be applied, for example, to arthropod pests directly and/or habitats of arthropod pests (e.g., plant bodies, animal bodies, or soil).

When the arthropod pest-controlling composition of the present invention is used for controlling pests in agriculture and forestry, the application amount is usually 1 to 10,000 g/ha, preferably 10 to 500 g/ha of the compound of the present invention. When the arthropod pest-controlling composition of the present invention is in the form of an emulsifiable concentrate, a wettable powder, a flowable formulation or a microcapsule formulation, it is usually used after dilution with water so as to contain 1 to 1,000 ppm of the compound of the present invention. When the arthropod pest-controlling composition of then present invention is in the form of a dust or a granule, it is usually used as it is. The arthropod post-controlling composition of the present invention may be sprayed directly to plants to be protected from arthropod pests. Soil can be treated with the arthropod pest-controlling composition of the present invention to control arthropod pests living in the soil. Seedbeds before planting or planting holes or plant feet in planting can be also treated with the arthropod pest-controlling composition of the present invention. A sheet formulation of the arthropod pest-controlling composition of the present invention may be applied by winding it around plants, disposing it in the vicinity of plants, laying it on the soil surface at the plant feet, or the like.

The arthropod pest-controlling composition of the present invention can be used in crop lands such as cultivated lands, paddy fields, lawns and orchards. The arthropod pest-controlling composition of the present invention may control harmful arthropods in a crop land without causing drug damage to crop plants cultivated in the crop land.

Examples of such crop plants include
Agricultural crops: corn, rice, wheat, barley, rye, oat, sorghum, cotton, soybean, peanut, sarrazin, sugar beet, rapeseed, sunflower, sugar cane, tobacco etc.;
Vegetables: Solanaceae vegetables (eggplant, tomato, green pepper, hot pepper, potato etc.), Cucurbitaceae vegetables (cucumber, pumpkin, zucchini, watermelon, melon etc.), Cruciferae vegetables (Japanese radish, turnip, horseradish, kohlrabi, Chinese cabbage, cabbage, brown mustard, broccoli, cauliflower etc.), Compositae vegetables (burdock, garland chrysanthemum, artichoke, lettuce etc.), Liliaceae vegetables (Welsh onion, onion, garlic, asparagus etc.), Umbelliferae vegetables (carrot, parsley, celery, parsnip etc.), Chenopodiaceae vegetables (spinach, Swiss chard etc.), Labiatae vegetables (Japanese basil, mint, basil etc.), strawberry, sweat potato, yam, aroid etc.;
Flowers;
Foliage plant;
Fruit trees: pomaceous fruits (apple, common pear, Japanese pear, Chinese quince, quince etc.), stone fleshy fruits (peach, plum, nectarine; Japanese plum, cherry, apricot, prune etc.), citrus plants (Satsuma mandarin, orange, lemon, lime, grapefruit etc.), nuts (chestnut, walnut, hazel nut, almond, pistachio, cashew nut, macadamia nut etc.), berry fruits (blueberry, cranberry, blackberry, raspberry etc.), grape, persimmon, olive, loquat, banana, coffee, date, coconut etc.;
Trees other than fruit trees: tea, mulberry, flowering trees and shrubs, street trees (ash tree, birch, dogwood, eucalyptus, ginkgo, lilac, maple tree, oak, poplar, cercis, Chinese sweet gum, plane tree, zelkova, Japanese arborvitae, fir tree, Japanese hemlock, needle juniper, pine, spruce, yew) etc.

The above-described crop plants include crop plants having resistance to herbicides such as HPPD inhibitors (e.g. isoxaflutole), ALS inhibitors (e.g. imazethapyr and thifensulfuron-methyl), EPSP synthesizing enzyme inhibitors, glutamine synthesizing enzyme inhibitors, acetyl CoA carboxylase inhibitors, and bromoxynil, which resistance is imparted by a classical breeding method or a genetic engineering technique.

Examples of the crop plant having herbicide resistance imparted by a classical breeding method include Clearfield (registered mark) canola resistant to an imidazolinone herbicide such as imazethapyr, and STS soybean resistant to a sulfonylurea ALS inhibitor herbicide such as thifensulfuron-methyl. Examples of the crop plant having resistance to an acetyl CoA carboxylase inhibitor such as a trione oxime herbicide or an aryloxy phenoxypropionic acid herbicide include SR corn. The crop plants having resistance to acetyl CoA carboxylase inhibitors are found in, for example, Proc. Natl. Acad. Sci. USA 1990, 87, p. 7175-7179. In addition, a mutant acetyl CoA carboxylase resistant to an acetyl CoA carboxylase inhibitor is known, for example, in Weed Science 53: p.728-746, 2005. When a gene encoding the mutant acetyl CoA carboxylase is introduced into a crop plant by a genetic engineering technique or when a mutation related to impartation of resistance is introduced into a gene encoding acetyl CoA carboxylase of a crop plant, a crop plant having the resistance to an acetyl CoA carboxylase inhibitor can be produced. Nucleic acids for introduction of a base substitution mutation' can be introduced into the cell of a crop plant by chimeraplasty (see, Gura T. 1999, Repairing the Genome's Spelling Mistakes, Science 285: 316-318) to induce a site-directed amino acid mutation in the gene targeting an acetyl CoA carboxylase inhibitor or herbicide of the crop plant, and thereby a crop plant resistant to an acetyl CoA carboxylase inhibitor or herbicide can be' produced.

Examples of the crop plant having herbicide resistance imparted by a genetic engineering technique include corn cultivars having resistance to glyphosate or glufosinate. Some of such corn cultivars are sold under the trade name of RoundupReady (registered mark), LibertyLink (registered mark), and the like.

The above-described crop plants include crop plants having an ability to produce an insecticidal toxin, for example a selective toxin originated from Bacillus, which ability is imparted by a genetic engineering technique.

Examples of insecticidal toxins produced in such genetically engineered plants include insecticidal proteins derived from Bacillus cereus and Bacillus popilliae; insecticidal proteins such as δ-endotoxins derived from Bacillus thuringiensis (e.g. Cry1Ab, Cry1Ac, Cry1F, Cry1Fa2, Cry2Ab, Cry3A, Cry3Bb1 and Cry9C), VIP 1, VIP 2, VIP 3 and VIP 3A; insecticidal proteins derived from nematodes; toxins produced by animals such as scorpion toxins, spider toxins, bee toxins and insect-specific nerve toxins; fungal toxins; plant lectin; agglutinin; protease inhibitors such as trypsin inhibitors, serine protease inhibitors, patatin, cystatin, and papain inhibitors; ribosome-inactivating proteins (RIP) such as ricin, corn-RIP, abrin, saporin, and briodin; steroid metabolizing enzymes such as 3-hydroxysteroid oxidase, ecdysteroid-UDP-glucosyltransferase, and cholesterol oxidase; ecdysone inhibitors; HMG-CoA reductase; ion channel inhibitors such as sodium channel inhibitors and calcium channel inhibitors; juvenile hormone esterase; diuretic hormone receptors; stilbene synthase; bibenzyl syntase; chitinase; and glucanase.

The toxins produced in such genetically engineered plants also include hybrid toxins, partly deficient toxins and modified toxins of insecticidal proteins such as δ-endotoxin proteins (e.g., Cry1Ab, Cry1Ac, Cry1F, Cry1Fa2, Cry2Ab, Cry3A; Cry3Bb1 and Cry9C), VIP1, VIP2, VIP3, and VIP3A. The hybrid toxin is made by combining different domains of the insecticidal proteins by a genetic engineering technique. An example of the partly deficient toxin includes Cry1Ab in which a part of amino acids is deleted. An example of the modified toxin includes a toxin in which one or more of amino acids of a naturally occurring toxin are substituted.

Examples of the insecticidal toxin and the genetically engineered crop plant having the ability to produce the insecticidal toxin are described, for example, in EP-A-0 374 753, WO 93/07278, WO 95/34656, EP-A-0 427 529, EP-A-451878, or WO 03/052073.

The genetically engineered crop plant having the ability to produce the insecticidal toxin particularly has resistance to attack by a coleopteran pest, dipteran pest or a lepidopteran pest.

Genetically engineered plants which have one or more pest-resistance genes and thereby produce one or more insecticidal toxins are also known, and some of them are commercially available. Examples of such genetically engineered plants include YieldGard (registered mark) (a corn cultivar expressing Cry1Ab toxin), YieldGard Rootworm (registered mark) (a corn cultivar expressing Cry3Bb1 toxin), YieldGard Plus (registered mark) (a corn cultivar expressing Cry1Ab and Cry3Bb1 toxins), Heculex I (registered mark) (a corn cultivar expressing Cry1Fa2 toxin and phosphinothricin N-acetyltransferase (PAT) for imparting resistance to gluphosinate), NuCOTN33B (registered mark) (a cotton cultivar expressing Cry1Ac toxin), Bollgard I (registered mark) (a cotton cultivar expressing Cry1Ac toxin), Bollgard II (registered mark) (a cotton cultivar expressing Cry1Ac and Cry2Ab toxins), VIPCOT (registered mark) (a cotton cultivar expressing VIP toxin), NewLeaf (registered mark) (a potato cultivar expressing Cry3A toxin), NatureGard Agrisure GT Advantage (registered mark) (GA21 glyphosate-resistance character), Agrisure CB Advantage (registered mark) (Bt11 corn borer (CB) character), and Protecta registered mark).

The above-described crop plants include those having an ability to produce an anti-pathogen substance which ability is imparted by a genetic engineering technique.

Examples of the anti-pathogen substance include PR proteins (PRPs, described in EP-A-0 392 225); ion channel inhibitors such as sodium channel inhibitors, and calcium channel inhibitors (e.g. KP1, KP4, or KP6 toxins produced by viruses); stilbene synthase; bibenzyl synthase; chitinase; glucanase; and substances produced by microorganisms such as peptide antibiotics, heterocycle-containing antibiotics, and protein factors involved in plant disease-resistance (described in WO 03/000906). Such anti-pathogen substances and genetically engineered plants which produce the anti-pathogen substances are described in EP-A-0 392 225, WO 05/33818, or EP-A-0 353 191.

When the arthropod pest-controlling composition of the present invention is used for control of epidemic, the application amount is usually 0.001 to 10 mg/m³ of the compound of the present invention for application to space, and 0.001 to 100 mg/m² of the compound of the present invention for application to a plane. When the arthropod pest-controlling composition of the present invention is in the form of an emulsifiable concentrate, a wettable powder or a flowable formulation, it is usually applied after dilution with water so as to contain usually 0.001 to 10,000 ppm of the compound of the present invention. When the arthropod pest-controlling composition of the present invention is in the form of an oil solution, an aerosol formulation, a smoking pesticide or a poison bait, it is usually applied as it is.

When the arthropod pest-controlling composition of the present invention is used for controlling external parasites of livestock such as a cow, a horse, a pig, a sheep, a goat and a chicken, or small animals such as a dog, a cat, a rat and a mouse, it can be applied to the animals by a known method in the veterinary field. Specifically, when systemic control is intended, the arthropod pest-controlling composition of the present invention is administered, for example, as a tablet, a mixture with feed, a suppository or an injection (e.g., intramuscularly, subcutaneously, intravenously, or intraperitoneally). When non-systemic control is intended, the arthropod pest-controlling composition of the present invention is applied to an animal by spraying, pour-on treatment or a spot-on treatment with the arthropod pest-controlling composition in the form of an oil solution or an aqueous liquid, by washing the animal with the arthropod pest-controlling composition in the form of a shampoo formulation, or by attaching a collar or a ear tag made of the arthropod pest-controlling composition in the form of a resin formulation to the animal. When the arthropod pest-controlling composition of the present invention is administered to an animal, the dose is usually in the range of 0.1 to 1,000 mg of the compound of the present invention per 1 kg body weight of the animal.

The arthropod pest-controlling composition of the present invention can be used in admixture or combination with other insecticides, nematocides, acaricides, fungicides, herbicides, plant growth regulators, synergists, fertilizers, soil conditioners, or animal feed.

Examples of the active ingredient of the insecticides include:
(1) organic phosphorus compounds:
   acephate, aluminium phosphide, butathiofos, cadusafos, chlorethoxyfos, chlorfenvinphos, chlorpyrifos, chlorpyrifos-methyl, cyanophos:CYAP, diazinon, DCIP(dichlorodiisopropyl ether), dichlofenthion:ECP, dichlorvos:DDVP, dimethoate, dimethylvinphos, disulfoton, EPN, ethion, ethoprophos, etrimfos, fenthion:MPP, fenitrothion:MEP, fosthiazate, formothion, hydrogen phosphide, isofenphos, isoxathion, malathion, mesulfenfos, methidathion:DMTP, monocrotophos, naled:BRP, oxydeprofos:ESP, parathion, phosalone, phosmet:PMP, pirimiphos-methyl, pyridafenthion, quinalphos, phenthoate:PAP, profenofos, propaphos, prothiofos, pyraclorfos, salithion, sulprofos, tebupirimfos, temephos, tetrachlorvinphos, terbufos, thiometon, trichlorphon:DEP, vamidothion, phorate, cadusafos, and the like;
(2) carbamate compounds:
   alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb:MIPC, metolcarb, methomyl, methiocarb, NAC, oxamyl, pirimicarb, propoxur:PHC, XMC, thiodicarb, xylylcarb, aldicarb, and the like;
(3) synthetic pyrethroid compounds:
   acrinathrin, allethrin, beta-cyfluthrin, bifenthrin, cycloprothrin, cyfluthrin, cyhalothrin, cypermethrin, empenthrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, gamma-cyhalothrin, furamethrin, tau-fluvalinate, metofluthrin; 2,3,5,6-tetrafluoro-4-methylbenzyl 2,2-dimethyl-3-(1-propenyl)cyclopropanecarboxylate, 2,3,5,6-tetrafluoro-4-(methoxymethyl)benzyl 2,2-dimethyl-3-(2-methyl-1-propenyl)cyclopropanecarboxylate, 2,3,5,6-tetrafluoro-4-(methoxymethyl)benzyl 2,2-dimethyl-3-(2-cyano-1-propenyl)cyclopropanecarboxylate, 2,3,5,6-tetrafluoro-4-(methoxymethyl)benzyl 2,2,3,3-tetramethylcyclopropanecarboxylate, and the like;
(4) nereistoxin compounds:
   cartap, bensultap, thiocyclam, monosultap, bisultap, and the like;
(5) neonicotinoid compounds:
   imidacloprid, nitenpyram, acetamiprid, thiamethoxam, thiacloprid, dinotefuran, clothianidin, and the like;
(6) benzoylurea compounds:
   chlorfluazuron, bistrifluron, diafenthiuron, diflubenzuron, fluazuron, flucycloxuron), flufenoxuron, hexaflumuron, lufenuron, novaluron, noviflumuron, teflubenzuron, triflumuron, triazuron, and the like;
(7) phenylpyrazole compounds:
   acetoprole, ethiprole, fipronil, vaniliprole, pyriprole, pyrafluprole, and the like;
(8) Bt toxin insecticides:
   live spores or crystal toxins originated from Bacillus thuringiesis and a mixture thereof;
(9) hydrazine compounds:
   chromafenozide, halofenozide, methoxyfenozide, tebufenozide, and the like;
(10) organic chlorine compounds:
   aldrin, dieldrin, dienochlor, endosulfan, methoxychlor, and the like;
(11) natural insecticides:
   machine oil, nicotine-sulfate, and the like;
(12) other insecticides:
   avermectin-B, bromopropylate, buprofezin, chlorphenapyr, cyromazine, D-D(1,3-Dichloropropene), emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, arsenic acid, benclothiaz, calcium cyanamide, calcium polysulfide, chlordane, DDT, DSP, flufenerim, flonicamid, flurimfen, formetanate, metam-ammonium, metam-sodium, methyl bromide, Potassium oleate, protrifenbute, spiromesifen, Sulfur, metaflumizone, spirotetramat, pyrifluquinazone, spinetoram, chlorantraniliprole, tralopyril, a compound represented by the following formula (A): 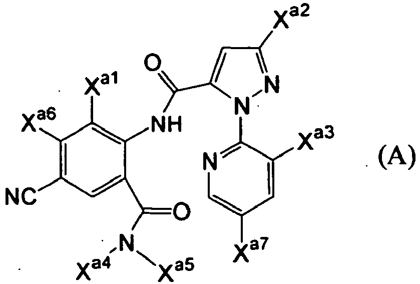 wherein X^{a1} represents a methyl group, a chlorine atom, a bromine atom, or a fluorine atom, X^{a2} represents a fluorine atom, a chlorine atom, a bromine atom, a C1-C4 haloalkyl group, or a C1-C4 haloalkoxy group, X^{a3} represents a fluorine atom, a chlorine atom, or a bromine atom, X^{a4} represents an optionally substituted C1-C4 alkyl group, an optionally substituted C3-C4 alkenyl group, an optionally substituted C3-C4 alkynyl group, an optionally substituted C3-C5 cycloalkylalkyl group, or a hydrogen atom, _{X}^{a5} represents a hydrogen atom or a methyl group, X^{a6} represents a hydrogen atom, a fluorine atom, or a chlorine atom, and X^{a7} represents a hydrogen atom, a fluorine atom, or a chlorine atom; a compound represented by the following formula (B): 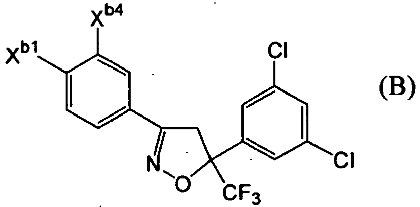 wherein X^{b1} represents a X^{b2} -NH-C (=O) group, a X^{b2}-C(=O)-NH-CH2 group, a X^{b3}-S(O) group, an optionally substituted pyrrol-1-yl group, an optionally substituted imidazol-1-yl group, an optionally substituted pyrazol-1-yl group, or an optionally substituted 1,2,4-triazol-1-yl group, X^{b2} represents an optionally substituted C1-C4 haloalkyl group such as a 2,2,2-trifluoroethyl group, or an optionally substituted C3-C6 cycloalkyl group such as a cyclopropyl group, X^{b3} represents an optionally substituted C1-C4 alkyl group such as a methyl group, and X^{b4} represents a hydrogen atom, a chlorine atom, a cyano group, or a methyl group; and a compound represented by the following formula (C): 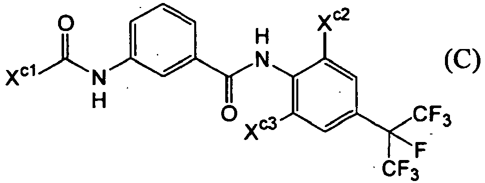 wherein X^{c1} represents an optionally substituted C1-C4 alkyl group such as a 3,3,3-trifluoropropyl group, an optionally substituted C1-C4 alkoxy group such as a 2,2,2-trichloroethoxy group, an optionally substituted phenyl group such as a 4-cyanophenyl group, or an optionally substituted pyridyl group such as a 2-chloro-3-pyridyl group, X^{c2} represents a methyl group or a trifluoromethylthio group, and X^{c3} represents a methyl group or a halogen atom.

Examples of the active ingredient of the acaricides include acequinocyl, amitraz, benzoximate, bifenazate, bromopropylate, chinomethionat, chlorobenzilate, CPCBS(chlorfenson), clofentezine, cyflumetofen, Kelthane (dicofol), etoxazole, fenbutatin oxide, fenothiocarb, fenpyroximate, fluacrypyrim, fluproxyfen, hexythiazox, propargite:BPPS, polynactins, pyridaben, Pyrimidifen, tebufenpyrad, tetradifon, spirodiclofen, spiromesifen, spirotetramat, amidoflumet, and cyenopyrafen.

Examples of the active ingredient of the nematocides include DCIP, fosthiazate, levamisol, methyisothiocyanate, morantel tartarate, and imicyafos.

Examples of the active ingredient of the fungicides include strobilurin compounds such as azoxystrobin; organophosphate compounds such as tolclofos-methyl; azole compounds such as triflumizole, pefurazoate and difenoconazole; fthalide, flutolanil, validamycin, probenazole, diclomezine, pencycuron, dazomet, kasugamycin, IBP, pyroquilon, oxolinic acid, tricyclazole, ferimzone, mepronil, EDDP, isoprothiolane, carpropamid, diclocymet, furametpyr, fludioxonil, procymidone and diethofencarb.

There is no limitation on the herbicides, plant growth regulators, synergists, fertilizers, soil conditioners or animal feed, and conventionally known herbicides, plant growth regulators, synergists, fertilizers, soil conditioners or animal feed can be used.

### Examples

Hereinafter, the present invention is described in more detail by way of Production Examples, Formulation Examples and Test Examples. However, the present invention is not limited to these Examples.

As used herein, abbreviations have the following meanings.
Me: methyl group, Et: ethyl group, Bn: benzyl group, Ph: phenyl group, Ts: p-toluenesulfonyl group, and Ac: acetyl group.

First, Production Examples of the compound of the present invention are shown.

### Reference Production Example 1

### [Step 1-1]

To a suspension of 10.28 g of potassium thioacetate in 80 ml of N,N-dimethylformamide was added dropwise 7.73 g of 1-chloro-2-methoxyethane under a nitrogen atmosphere at 0°C over 15 minutes, followed by stirring at room temperature for 1 hour. After the reaction mixture was heated to 60°C, stirred for 4 hours and then cooled to room temperature, 100 ml of ethyl acetate and 100 ml of an aqueous 1 N hydrochloric acid solution were sequentially added thereto. An organic layer was separated from the mixture. An aqueous layer was extracted twice with 100 ml of ethyl acetate. Organic layers were combined, washed sequentially with 30 ml of an aqueous 1 N hydrochloric acid solution, 100 ml of an aqueous saturated sodium hydrogen carbonate solution and then 100 ml of saturated brine, dried over sodium sulfate, filtered, and concentrated under reduced pressure. The residue was subjected to silica gel column chromatography to obtain 10.12 g of S-(2-methoxyethyl) thioacetate represented by the following formula. 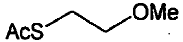 ¹H-NMR (CDCl₃, TMS, δ (ppm)): 2.34 (3H, s), 3.09 (2H, t), 3.37 (3H, s), 3.52 (2H, t)

### [Step 1-2]

A solution of 10.12 g'of S-(2-methoxyethyl) thioacetate in 80 ml of tetrahydrofuran was cooled to 0°C. To the solution was added dropwise 15.62 g of a 28% solution of sodium methoxide over 15 minutes, followed by stirring at room temperature for 1 hour. To the mixture, 6.07 g of chloroacetonitrile was added at 0°C, followed by stirring at room temperature for 4 hours. The reaction misture was cooled in an ice bath, and 100 ml of a saturated aqueous sodium chloride solution was added thereto. Then the mixture was extracted twice with 100 ml of t-butyl methyl ether. Organic layers were combined, washed with a saturated aqueous sodium chloride solution, dried over sodium sulfate, filtered and then concentrated under reduced pressure. The residue was subjected to silica gel column chromatography to obtain 4.10 g of (2-methoxyethylthio)acetonitrile represented by the following formula. 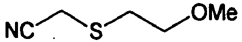 ¹H-NMR (CDCl₃, TMS, δ (ppm)): 2.92 (2H, t), 3.39 (3H, s), 3.43 (2H, s), 3.70 (2H, t)

### [Step 1-3]

To a suspension of 19.16 g of a double salt of 2KHSO₅ • KHSO₄ • K₂SO₄ (Oxone (registered mark)) in 60 ml of water was added dropwise a solution of 4.10 g of (2-methoxyethylthio)acetonitrile in 60 ml of methanol under a nitrogen atmosphere at 0°C over 30 minutes, followed by stirring at room'temperature for 1 hour. The reaction mixture was heated to 50°C, followed by stirring for 2 hours. After the reaction mixture was cooled in an ice bath, 80 ml of an aqueous 10% sodium sulfite solution was added thereto. After metanol was disstilled off from the mixture under reduced pressure, the mixture was extracted twice with 100 ml of ethyl acetate. Organic layers were combined, washed with 40 ml of an aqueous 10% sodium sulfite solution and then 50 ml of a saturated aqueous sodium chloride solution, dried over sodium sulfate, filtered, and then concentrated under reduced pressure. The residue was subjected to silica gel column chromatography to obtain 2.88 g of (2-methoxyethanesulfonyl)acetonitrile represented by the following formula. 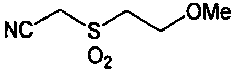 ¹H-NMR (CDCl₃, TMS, δ (ppm)): 3.43 (3H, s), 3.45 (2H, t), 3.86 (2H, t), 4.13 (2H, s)

### Production Example 1.

To a solution of 1.63 g of (2-methoxyethanesulfonyl)acetonitrile obtained in Reference Production Example 1 in 10 ml of N,N-dimethylformamide were added 1.38 g of potassium carbonate and 2.74 g of 1-iodo-3,3,4,4,4-pentafluorobutane, followed by stirring at room temperature for 1 hour. The reaction mixture was heated to 70°C and stirred for 4 hours. After the reaction mixture was cooled to room temperature, 30 ml of ethyl acetate, 30 ml of an aqueous 1 N hydrochloric acid solution were sequentially added. An organic layer was separated from the mixture and an aqueous layer was extracted twice with 30 ml of ethyl acetate. Organic layers were combined, washed sequentially with 30 ml of an aqueous 1 N hydrochloric acid solution, 30 ml of an aqueous saturated sodium hydrogen carbonate solution and then 30 ml of a saturated aqueous sodium chloride solution, dried over sodium sulfate, filtered, and then concentrated under reduced pressure. The residue was subjected to silica gel column chromatography to obtain 0.89 g of 5,5,6,6,6-pentafluoro-2-(2-methoxyethanesulfonyl)hexanenitrile (hereinafter referred to as the present compound (1)) represented by the following formula. 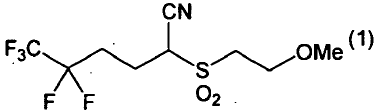 ¹H-NMR (CDCl₃, TMS, δ (ppm)): 2.27-2.60 (4H, m), 3.20-3.27 (1H, m), 3.45 (3H, s), 3.74-3.85 (2H, m), 3.90-3.97 (1H, m), 4.37-4.42 (1H, m)

### Reference Production Example 2

### [Step 2-1]

To a solution of 11.17 g of (3-methoxypropyl) p-toluenesulfonate in 25 ml of N,N-dimethylformamide was added 5.88 g of potassium thioacetate under a nitrogen atmosphere at room temperature, followed by stirring for 2 hours. The reaction mixture was heatede to 50°C, stirred for 30 minutes and then cooled to room temperature. To the mixture were added sequentially 40 ml of ethyl acetate and then 70 ml of an aqueous 1 N hydrochloric acid solution. An organic layer was separated and an aqueous layer was extracted three times with 40 ml of ethyl acetate. Organic layers were combined, washed sequentially with 100 ml of an aqueous 1 N hydrochloric acid solution, 30 ml of an aqueous saturated sodium hydrogen carbonate solution and then 30 ml of a saturated aqueous sodium chloride solution, dried over sodium sulfate, filtered, and then concentrated under reduced pressure. The residue was subjected to silica gel column chromatography to obtain 6.06 g of a S-(3-methoxypropyl) thioacetate represented by the following formula. 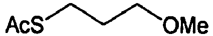 ¹H-NMR (CDCl₃, TMS, δ (ppm)): 1.84 (2H, tt), 2.33 (3H, s), 2.95 (2H, t), 3.33 (3H, s), 3.42 (2H, t)

### [Step 2-2]

A solution of 6.06 g of S-(3-methoxypropyl) thioacetate in 40 ml of tetrahydrofuran was cooled to 0°C. Thereto was added dropwise 7.87.g of a 28% solution of sodium methoxide in methanol over 15 minutes, followed by stirring at room temperature for 30 minutes. To the mixture, 3.08 g of chloroacetonitrile was added at 0°C, followed by stirring at room temperature for 18 hours. After the reaction mixture was cooled in an ice bath, 50 ml of a saturated aqueous sodium chloride solution was added thereto. The mixture was extracted three times with 50 ml of ethyl acetate. Organic layers were combined, washed with 50 ml of a saturated aqueous sodium chloride solution, dried over sodium sulfate, filtered, and then concentrated under reduced pressure. The residue was subjected to silica gel column chromatography to obtain 4.47 g of (3-methoxypropylthio)acetonitrile represented by the following formula. 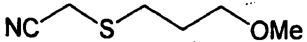 ¹H-NMR (CDCl₃, TMS, δ (ppm)): 1.93 (2H, tt), 2.84 (2H, t), 3.30 (2H, s), 3.35 (3H, s), 3.49 (2H, t)

### [Step 2-3]

To a suspension of 22.65 g of a double salt of 2KHSO₅ • KHSO₄ • K₂SO₄ (Oxone (registered mark)) in 90 ml of water was added dropwise a solution of 4.47 g of (3-methoxypropylthio)acetonitrile in 50 ml of methanol under a nitrogen atmosphere at 0°C over 30 minutes, followed by stirring at room temperature for 3 hours. The reaction mixture was cooled in an ice bath and 50 ml of an aqueous 10% sodium sulfite solution was added thereto. After methanol was distilled off from the mixure under reduced pressure, the mixture was extracted three times with 100 ml of ethyl acetate. Organic layers were combined, washed with 20 ml of an aqueous 10% sodium sulfite solution and then 50 ml of a saturated aqueous sodium chloride solution, dried over sodium sulfate, filtered, and then concentrated under reduced pressure. The residue was subjected to silica gel column chromatography to obtain 4.92 g of (3-methoxypropanesulfonyl) acetonitrile represented by the following formula. 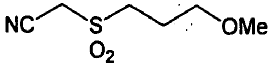 ¹H-NMR (CDCl₃, TMS, δ (ppm)): 2.18 (2H, tt), 3.37 (3H, s), 3.41 (2H, t), 3.54 (2H, t), 4.03 (2H, s)

### Production Example 2

To a solution of 0.50 g of (3-methoxypropanesulfonyl)acetonitrile obtained in Reference Production Example 2 in 6 ml of N,N-dimethylformamide were added 0.43 g of potassium carbonate and 0.85 g of 1-iodo-3,3,4,4,4-pentafluorobutane, followed by stirring at room temperature for 2 hours. The reaction mixture was heated to 50°C and stirred for 2 hours and half. After the reaction mixture was cooled to room temperature, 30 ml of ethyl acetate and then 10 ml of an aqueous 1 N hydrochloric acid solution were sequentially added thereto. An organic layer was separated from the mixture, and an aqueous layer was extracted twice with 30 ml of ethyl acetate. Organic layers were combined, washed sequentially with 10 ml of an aqueous 1 N hydrochloric acid solution, 10 m of an aqueous saturated sodium hydrogen carbonate solution and then 10 ml of a saturated aqueous sodium chloride solution, dried over sodium sulfate, filtered, and then concentrated under reduced pressure. The residue was subjected to silica gel column chromatography to obtain 0.69 g of 5,5,6,6,6-pentafluoro-2-(3-methoxypropanesulfonyl)hexanenitrile (hereinafter referred to as the present compound (2)) represented by the following formula. 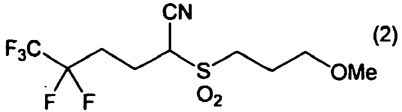 ¹H-NMR (CDCl₃, TMS, δ (ppm)): 2.21 (2H, tt), 2.43 (4H, m), 3.37 (3H, s), 3.42-3.46 (2H, m), 3.49-3.59 (2H, m), 4.09-4.12 (1H, m)

### Reference Production Example 3

### [Step 3-1]

To a solution of 36.25 g of (2-isopropoxyethyl) p-toluenesulfonate in 70 ml of N,N-dimethylformamide vas added 16.82 g of potassium thioacetate under a nitrogen atmosphere at room temperature, followed by stirring for 1 hour. The reaction mixture was heated to 60°C, stirred for 4 hours, and cooled to room temperature. Thereto, 150 ml of ethyl acetate and then 300 ml of an aqueous 1 N hydrochloric acid solution were added. An organic.layer was separated from the mixture and an aqueous layer was extracted twice with 150 ml of ethyl acetate. Organic layers were combined, washed sequentially with 100 ml of an aqueous 1 N hydrochloric acid solution, 100 ml of an aqueous saturated sodium hydrogen carbonate solution and then 100 ml of a saturated aqueous sodium chloride solution, dried over sodium sulfate, filtered, and then concentrated under reduced pressure. The residue was subjected to silica gel column chromatography to obtain 13.56 g of a S-(2-isopropoxyethyl) thioacetate represented by the following formula. 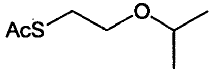 ¹H-NMR (CDCl₃, TMS, δ (ppm)): 1.15 (6H, d), 2.34 (3H, s), 3.07 (2H, t), 3.54 (2H, t), 3.60 (1H, qq),

### [Step 3-2]

A solution of 13.56 g of S-(2-isopropoxyethyl) thioacetate in 80 ml of tetrahydrofuran was cooled to 0°C, and 16.01 g of a 28% solution of sodium methoxide in methanol was added dropwise thereto over 15 minutes, followed by stirring at room temperature for 1 hour. To the mixture, 6.27 g of chloroacetonitrile was added at 0°C, followed by stirring at room temperature for 4 hours. After the reaction mixture was cooled in an ice bath, a saturated aqueous sodium chloride solution was added thereto. Then, the mixture was extracted twice with 100 ml of t-butyl methyl ether. Organic layers were combined, washed with a saturated aqueous sodium chloride solution, dried over sodium sulfate, filtered, and then concentrated under reduced pressure. The residue was subjected to silica gel column chromatography to obtain 12.39 g of (2-isopropoxyethylthio)acetonitrile represented by the following formula. 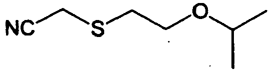 ¹H-NMR (CDCl₃, TMS, δ (ppm)): 1.18 (6H, d), 2.90 (2H, t), 3.47 (2H, s), 3.60 (1H, qq), 3.72 (2H, t)

### [Step 3-3]

To a suspension of 51.00 g of a double salt of 2KHSO₅ • KHSO₄ • K₂SO₄ (Oxone (registered mark)) in 80 ml of water was added dropwise a solution of 12.39 g of (2-isopropoxyethylthio)acetonitrile in 80 ml of methanol under a nitrogen atmosphere at 0°C over 30 minutes, followed by stirring at room temperature for 1 hour. The reaction mixture was heated to 50°C and stirred for 2 hours. After the reaction mixture was cooled in an ice bath, 100 ml of an aqueous 10% sodium sulfite solution was added thereto. After methanol was distilled off from the mixure under reduced pressure, the mixture was extracted twice with 100 ml of ethyl acetate. Organic layers were combined, washed with 50 ml of an aqueous 10% sodium sulfite solution and then 50 ml of a saturated aqueous sodium chloride solution, dried over sodium sulfate, filtered, and then concentrated under reduced pressure. The residue was subjected to silica gel column chromatography to obtain to obtain 14.13 g of (2-isopropoxyethanesulfonyl)acetonitrile represented by the following formula. 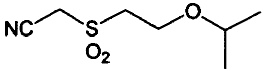 ¹H-NMR (CDCl₃, TMS, δ (ppm)): 1.20 (6H, d), 3.44 (2H, t), 3.69 (1H, qq), 3.88 (2H, t), 4.17 (2H, s)

### Production Example 3

To a solution of 1.91 g of (2-isopropoxyethanesulfonyl)acetonitrile obtained in Reference Production Example 3 in 10 ml of N,N-dimethylformamide were added 1.38 g of potassium carbonate and 2.74 g of 1-iodo-3,3,4,4,4-pentafluorobutanewere, followed by stirring at room temperature for 1 hour. The reaction mixture was heated to 70°C and stirred for 4 hours. After the reaction mixture was cooled to room temperature, 30 ml of ethyl acetate and then 30 ml of an aqueous 1 N hydrochloric acid solution were sequentially added thereto. An organic layer was separated and an aqueous layer was extracted twice with 30 ml of ethyl acetate. Organic layers were combined, washed sequentially with 30 ml of an aqueous 1 N hydrochloric acid solution, 30 ml of an aqueous saturated sodium hydrogen carbonate solution and then 30 ml of a saturated aqueous sodium chloride solution, dried over sodium sulfate, filtered, and then concentrated under reduced pressure. The residue was subjected to silica gel column chromatography to obtain 1.03 g of 5,5,6,6,6-pentafluoro-2-(2-isopropoxyethanesulfonyl)hexanenitrile (hereinafter referred to as the present compound (3)) represented by the following formula. 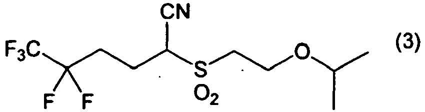 ¹H-NMR (CDCl₃, TMS, δ (ppm)): 1.17 (3H, d), 1.19 (3H, d), 2.26-2.61 (4H, m), 3.20-3.26 (1H, m), 3.62-3.72 (1H, m), 3.75-3.85 (2H, m), 3.92-3.99 (1H, m), 4.52-4.58 (1H, m)

### Reference Production Example 4

### [Step 4-1]

To a solution of 11.72 g of 2-methoxy-2-methyl-1-propanol in 50 ml of pyridine was added 21.49 g of p-toluenesulfonyl chloride, followed by stirring at room temperature overnight. To the reaction mixture, 150 ml of ethyl acetate and then 150 ml of an aqueous 1 N hydrochloric acid solution were sequentially added. An organic layer was separated from the mixture and an aqueous layer was extracted twice with 100 ml of ethyl acetate. Organic layers were combined, washed sequentially with 50 ml of an aqueous 1 N hydrochloric acid solution, 100 ml of an aqueous saturated sodium hydrogen carbonate solution and then 100 ml of a saturated aqueous sodium chloride solution, dried over sodium sulfate, filtered, and then concentrated under reduced pressure. The residue was subjected to silica gel column chromatography to obtain 26.14 g of (2-methoxy-2-methylpropyl) p-toluenesulfonate represented by the following formula. 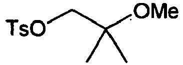 ¹H-NMR (CDCl₃, TMS, δ (ppm)): 1.15 (6H, s), 2.46 (3H, s), 3.16 (3H, s), 3.85 (2H, s), 7.34 (2H, d), 7.80 (2H, d)

### [Step 4-2]

To a suspension of 11.56 g of potassium thioacetate in 100 ml of N,N-dimethylformamide was added 26.14 g of (2-methoxy-2-methylpropyl) p-toluenesulfonate under a nitrogen atmosphere, followed by stirring at room temperature for 1 hour. The reaction mixture was heated to 70°C, stirred for 48 hours, and then cooled to room temperature. Thereto were added 200 ml of methyl tert-butyl ether (hereinafter refrred to as MTBE) and then 100 ml of an aqueous 1 N hydrochloric acid solution. An organic layer was separated from the mixture and an aqueous layer was extracted twice with 100 ml of MTBE. Organic layers were combined, washed' sequentially with 30 ml of an aqueous 1 N hydrochloric acid solution, 100 ml of an aqueous saturated sodium hydrogen carbonate solution and then 100 ml of a saturated aqueous sodium chloride solution, dried over sodium sulfate, filtered, and then concentrated under reduced pressure. The residue was subjected to silica gel column chromatography to obtain 8.92 g of S-(2-methoxy-2-methylpropyl) thioacetate represented by the following formula. 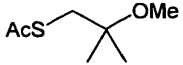 ¹H-NMR (CDCl₃, TMS, δ (ppm)): 1.21 (6H, s), 2.37 (3H, s), 3.12 (2H, s), 3.19 (3H, s)

### [Step 4-3]

A solution of 8.92 g of S-(2-methoxy-2-methylpropyl) thioacetate in 55 ml of tetrahydrofuran was cooled to 0°C. Thereto was added dropwise 10.61 g of 28% solution of sodium methoxide in methanol over 15 minutes, followed by stirring at room temperature for 1 hour. To the mixture, 4.15 g of chloroacetonitrile was added at 0°C, followed by stirring at room temperature for 4 hours. The reaction mixture was cooled in an ice bath, and 50 ml of a saturated aqueous sodium chloride solution was added thereto. Then the mixture was extracted twice with 50 ml of ethyl acetate. Organic layers were combined, washed with 50 ml of a saturated aqueous sodium chloride solution, dried over sodium sulfate, filtered, and then concentrated under reduced pressure. The residue was subjected to silica gel column chromatography to obtain 8.25 g of (2-methoxy-2-methylpropylthio)acetonitrile represented by the following formula. 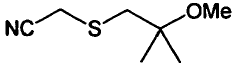 ¹H-NMR (CDCl₃, TMS, δ (ppm)): 1.29 (6H, s), 2.83 (2H, s), 3.22 (3H, s), 3.41 (2H, s)

### [Step 4-4]

To a suspension of 37.15 g of a double salt of 2KHSO₅ • KHSO₄ • K₂SO₄ (Oxone (registered mark)) in 120 ml of water were added dropwise a solution of 8.25 g of (2-methoxy-2-methylpropylthio)acetonitrile in 120 ml of methanol under a nitrogen atmosphere at 0°C over 30 minutes, followed by stirring at room temperature for 4 hours. The reaction mixture was heated to 50°C and stirred for 1 hour.. The reaction mixture was cooled in an ice bath and 80 ml of an aqueous 10% sodium sulfite solution was added thereto. After metanol was distilled off from the mixture under reduced pressure, the mixture was extracted three times with 100 ml of ethyl acetate. Organic layers were combined, washed with 40 ml of an aqueous 10% sodium sulfite solution and then 50 ml of a saturated aqueous sodium chloride solution, dried over sodium sulfate, filtered, and then concentrated under reduced pressure. The residue was subjected to silica gel column chromatography to obtain 9.18 g of (2-methoxy-2-methylpropanesulfonyl)acetonitrile represented by the following formula. 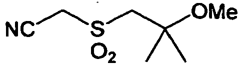 ¹H-NMR (CDCl₃, TMS, δ (ppm)): 1.43 (6H, s), 3.30 (3H, s), 3.38 (2H, s), 4.15 (2H, s)

### Production Example 4

To a solution of 0.96 g of (2-methoxy-2-methylpropanesulfonyl)acetonitrile obtained in Reference Production Example 4 in 5 ml of N,N-dimethylformamide were added 0.69 g of potassium carbonate and 1.37 g of 1-iodo-3,3,4,4,4-pentafluorobutane to the mixture, followed by stirring at room temperature for 1 hour. The reaction mixture was heated to 70°C and stirred for 4 hours. The mixture was cooled to room temperature, and 30 ml of ethyl acetate and then 30 ml of an aqueous 1 N hydrochloric acid solution were sequentially added thereto. An organic layer was separated from the mixture and an aqueous layer was extracted twice with 30 ml of ethyl acetate. Organic layers were combined, washed sequentially with 30 ml of an aqueous 1 N hydrochloric acid solution, 30 ml of an aqueous saturated sodium hydrogen carbonate solution and then 30 ml of a saturated aqueous sodium chloride solution, dried over sodium sulfate, filtered, and then concentrated under reduced pressure. The residue was subjected to silica gel column chromatography to obtain 0.87 g of 5;5,6,6,6-pentafluoro-2-(2-methoxy-2-methylpropanesulfonyl)hexanenitrile (hereinafter referred to as the present compound (4)) represented by the following formula. 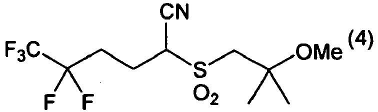 ¹H-NMR (CDCl₃, TMS, δ (ppm)): 1.37 (3H, s), 1.48 (3H, s), 2.29-2.56 (4H, m), 3.16 (1H, d), 3.28 (3H, s), 3.69 (1H, d), 4.51 (1H, t)

### Reference Production Example 5

### [Step 5-1]

A solution of 30.44 g of 2-mercaptoethanol in 200 ml of tetrahydrofuran was cooled to 0°C, and 75.24 g of a 28% solution of sodium methoxide in methanol was added dropwise thereto over 30 minutes, followed by stirring at room temperature for 1 hour. To the mixture, 29.22 g of chloroacetonitrile was added at 0°C, followed by stirring at room temperature overnight. The reaction mixture was cooled in an ice bath, and 50 ml of a saturated aqueous sodium chloride solution was added thereto. The mixture was extracted three times with 100 ml of ethyl acetate. Organic layers were combined, washed with 100 ml of a saturated aqueous sodium chloride solution, dried over sodium sulfate, filtered, and then concentrated under reduced pressure. The residue was subjected to silica gel column chromatography to obtain 45.00 g of (2-hydroxyethylthio)acetonitrile represented by the following formula. 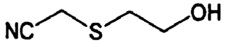 ¹H-NMR (CDCl₃, TMS, δ (ppm)): 2.95 (2Ht), 3.42 (2H, s), 3.93 (2H, t)

### [Step 5-2]

To a suspension of 33.77 g of a double salt of 2KHSO₅ • KHSO₄ • K₂SO₄ (Oxone (registered mark)) in 100 ml of water was added dropwise a solution of 5.86 g of (2-hydroxyethylthio)acetonitrile in 100 ml of methanol under a nitrogen atmosphere at 0°C over 30 minutes, followed by stirring at room temperature for 4 hours. The reaction mixture was heated to 50°C and stirred for 1 hour. A mixture was cooled in an ice bath, and 60 ml of an aqueous 10% sodium sulfite solution was added thereto. Then, methanol was distilled off under reduced pressure, and the mixture was extracted three times with 100 ml of ethyl acetate. Organic layers were combined, washed with 30 ml of an aqueous 10% sodium sulfite solution and then 50 ml of a saturated aqueous sodium chloride solution, dried over sodium sulfate, filtered, and then concentrated under reduced pressure. The residue was subjected to silica gel column chromatography to obtain 4.16 g of (2-hydroxyethanesulfonyl)acetonitrile represented by the following formula. 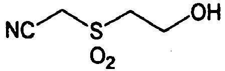 ¹H-NMR (CDCl₃, TMS, δ (ppm)) 3.48 (2Ht), 4.20 (2H, s), 4.21 (2H, t)

### Production Example 5

To a solution of 4.16 g of (2-hydroxyethanesulfonyl)acetonitrile obtained in Reference Production Example 5 in 30 ml of N,N-dimethylformamide were added 3.85 g of potassium carbonate and 7.64 g of 1-iodo-3,3,4,4,4-pentafluorobutane, followed by stirring at room temperature for 4 hours. The reaction mixture was heated to 50°C and stirred for 5 hours. The mixture was cooled to room temperature, and 150 ml of ethyl acetate and then 150 ml of an aqueous 1 N hydrochloric acid solution were sequentially added thereto. An organic layer was separated from the mixture and an aqueous layer was extracted twice with 100 ml of ethyl acetate. Organic layers were combined, washed sequentially with 50 ml of an aqueous 1 N hydrochloric acid solution, 50 ml of an aqueous saturated sodium hydrogen carbonate solution and then 50 ml of saturated brine, dried over sodium sulfate, filtered, and then concentrated under reduced pressure. The residue was subjected to silica gel column chromatography to obtain 2.36 g of 5,5,6,6,6-pentafluoro-2-(2-hydroxyethanesulfonyl)hexanenitrile (hereinafter referred to as the present compound (5)) represented by the following formula. 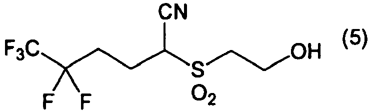 ¹H-NMR (CDCl₃, TMS, δ (ppm)): 2.23-2.57 (5H, m), 3.25-3.33 (1H, m), 3.75-3.83 (1H, m), 4.16-4.30 (2H, m), 4.44-4.48 (1H, m)

### Production Example 6

To a solution of 1.48 g of the present compound (5) in 5 ml of N,N-dimethylformamide were added 1.03 g of potassium carbonate and 1.06 g of iodomethane, followed by stirring at room temperature for 1 hour. The reaction mixture was heated to 70°C and stirred for 4 hours. The mixture was cooled to room temperature, and 30 ml of ethyl acetate and then 30 ml of an aqueous 1 N hydrochloric acid solution were sequentially added thereto. An organic layer was separated and an aqueous layer was extracted twice.with 30 ml of ethyl acetate. Organic layers were combined; washed sequentially with 30 ml of an aqueous 1 N hydrochloric acid solution, 30 ml of an aqueous saturated sodium hydrogen carbonate solution and then 30 ml of saturated brine, dried over sodium sulfate, filtered, and then concentrated under reduced pressure. The residue was subjected to silica gel column chromatography to obtain 1.18 g of 5,5,6,6,6-pentafluoro-2-(2-hydroxyethanesulfonyl)-2-methylhexanenitrile (hereinafter referred to as the present compound (6)) represented by the following formula. 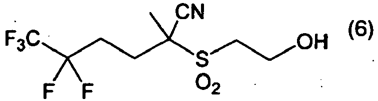 ¹H-NMR (CDCl₃, TMS, δ (ppm)): 1.82 (3H, s), 2.14-2.61 (5H, m), 3.47-3.64 (2H, m), 4.23-4.32 (2H, m) .

### Production Example 7

### [Step 7-1]

A solution of 3.36 g of the present compound (6) in 20 ml of thionyl chloride was heated under reflux for 6 hours. The reaction mixture was cooled to room temperature, and thionyl chloride was distilled off under reduced pressure. To the residue was added 20 ml of hexane and concentrated under reduced pressure, and such an operation was repeated twice. The residue was subjected to silica gel column chromatography to obtain 3.52 g of 5,5,6,6,6-pentafluoro-2-(2-chloroethanesulfonyl)-2-methylhexanenitrile represented by the following formula. 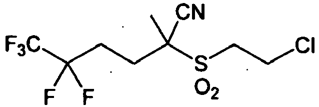 ¹H-NMR (CDCl₃, TMS, δ (ppm)): 1.81 (3H, s), 2.14-2.27 (4H, m), 3.62-3.76 (2H, m), 4.57-4.71 (2H, m)

### [Step 7-2]

To a solution of 0.30 g of 5,5,6,6,6-pentafluoro-2-(2-chloroethanesulfonyl)-2-methylhexanenitrile in 1 ml of N,N-dimethylformamide was added 0.10 g of sodium ethanethiolate, followed by stirring at room temperature for 4 hours. Thereto 20 ml of ethyl acetate and then 20 ml of an aqueous 1 N hydrochloric acid solution were sequentially added. An organic layer was separated and an aqueous layer was extracted twice with 20 ml of ethyl acetate. Organic layers were combined, washed sequentially with 20 ml of an aqueous 1 N hydrochloric acid solution, 20 ml of an aqueous saturated sodium hydrogen carbonate solution and then 20 ml of a saturated aqueous sodium chloride solution, dried over sodium sulfate, filtered, and then concentrated under reduced pressure. The residue was subjected to silica gel column chromatography to obtain 0.12 g of 5,5,6,6,6-pentafluoro-2-(2-ethylthioethanesulfonyl)-2-methylhexanenitrile (hereinafter referred to as the present compound (7)) represented by the following formula. 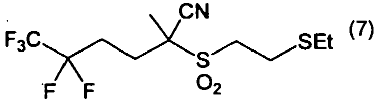 ¹H-NMR (CDCl₃, TMS, δ (ppm)): 1.32 (3H, t), 1.80 (3H, s), 2.15-2.55 (4H, m), 2.64 (2H, q), 3.02-3.11 (2H, m), 3.44-3.61 (2H, m)

Specific examples of the compound of the present invention are shown below.

A compound represented by the formula (Iα): 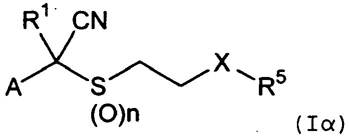 wherein A, X, R¹, R⁵ and n represent any one of combinations shown below. Combinations of A, X, R¹, R⁵ and n are as follows:
[branch number: A, X, R¹, R⁵, n] =
[1: CF₃CH₂CH₂, O, H, Me, 0];
[2: CF₃CF₂CF₂CH₂, O, H, Me, 0];
[3: CF₃CF₂CH₂CH₂, O, H, Me, 0];
[4: CF₃CH₂CH₂CH₂, O, H, Me, 0];
[5: CF₃CF₂CF₂CH₂CH₂, O, H, Me, 0];
[6: CF₃CF₂CH₂C₂CH₂, O, H, Me, 0];
[7: CF₃CH₂CH₂CH₂CH₂, O, H, Me, 0];
[8: CF₂HCF₂CF₂CF₂CH₂, O, H, Me, 0];
[9: CClF₂CClFCH₂CH₂, O, H, Me, 0];
[10: CBrF₂CF₂CH₂CH₂, O, H, Me, 0];
[11: CF₃CH(Me)CH₂, O, H, Me, 0];
[12: CF₃CH(Me)CH₂CH₂, O, H, Me, 0];
[13: CF₃CF₂CH(Me)CH₂CH₂, O, H, Me, 0];
[14: CF₃CH(Et)CH₂CH₂, O, H, Me, 0];
[15: CF₃CH(Pr)CH₂CH₂, O, H, Me, 0];
[16: CF₃CH(iPr)CH₂CH₂, O, H, Me, 0];
[17: CF₃CH(tBu)CH₂CH₂, O, H, Me, 0];
[18: CF₃CH(OMe)CH₂CH₂, O, H, Me, 0];
[19: CF₃CH(SMe)CH₂CH₂, O, H, Me, 0];
[20: CF₃CH₂CH₂, O, H, Me, 1];
[21: CF₃CF₂CF₂CH₂ , O, H, Me, 1];
[22: CF₃CF₂CH₂CH₂ , O, H, Me, 1];
[23: CF₃CH₂CH₂CH₂, O, H, Me, 1];
[24: CF₃CF₂CF₂CH₂ CH₂, O, H, Me, 1];
[25: CF₃CF₂CH₂CH₂CH₂, O, H, Me, 1];
[26: CF₃CH₂CH₂CH₂CH₂, O, H, Me, 1];
[27: CF₂HCF₂CF₂CF₂CH₂, O, H, Me, 1];
[28: CClF₂CClFCH₂CH₂, O, H, Me, 1];
[29: CBrF₂CF₂CH₂CH₂, O, H, Me, 1];
[30: CF₃CH(Me)CH₂, O, H, Me, 1];
[31: CF₃CH(Me)CH₂CH₂, O, H, Me, 1];
[32: CF₃CF₂CH(Me)CH₂CH₂, O, H, Me, 1];
[33: CF₃CH(Et)CH₂CH₂, O, H, Me, 1];
[34: CF₃CH(Pr)CH₂CH₂, O, H, Me, 1];
[35: CF₃CH(iPr)CH₂CH₂, O, H, Me, 1];
[36: CF₃CH(tBu)CH₂CH₂, O, H, Me, 1];
[37: CF₃CH(OMe)CH₂CH₂, O, H, Me, 1];
[38: CF₃CH(SMe)CH₂CH₂, O, H, Me; 1];
[39: CF₃CH₂CH₂, O, H, Me, 2];
[40: CF₃CF₂CF₂CH₂, O, H, Me, 2];
[41: CF₃CF₂CH₂CH₂, O, H, Me, 2];
[42: CF₃CH₂CH₂CH₂, O, H, Me, 2];
[43: CF₃CF₂CF₂CH₂CH₂, O, H, Me, 2] ;
[44: CF₃CF₂CH₂CH₂CH₂, O, H, Me, 2];
[45: CF₃CH₂CH₂CH₂CH₂, O, H, Me, 2];
[46: CF₂HCF₂CF₂CF₂CH₂, O, H, Me, 2];
[47: CClF₂CClFCH₂CH₂, O, H, Me, 2];
[48: CBrF₂CF₂CH₂CH₂, O, H, Me, 2];
[49: CF₃CH(Me)CH₂, O, H, Me, 2] ;
[50: CF₃CH(Me)CH₂CH₂, O, H, Me, 2];
[51: CF₃CF₂CH(Me)CH₂CH₂, O, H, Me, 2];
[52: CF₃CH(Et)CH₂CH₂, O, H, Me, 2];
[53: CF₃CH(Pr)CH₂CH₂, O, H, Me, 2];
[54: CF₃CH(iPr)CH₂CH₂, O, H, Me, 2];
[55: CF₃CH(tBu)CH₂CH₂, O, H, Me, 2];
[56: CF₃CH(OMe)CH₂CH2, O, H, Me, 2];
[57: CF₃CH(SMe)CH₂CH₂, O, H, Me, 2];
[58: CF₃CH₂CH₂, S, H, Me, 2];
[59: CF₃CF₂CF₂CH₂, S, H, Me, 2];
[60: CF₃CF₂CH₂CH₂, S, H, Me, 2];
[61: CF₃CH₂CH₂CH₂, S, H, Me, 2];
[62: CF₃CF₂CF₂CH₂CH₂, S, H, Me, 2];
[63: CF₃CF₂CH₂CH₂CH₂, S, H, Me, 2];
[64: CF₃CH₂CH₂CH₂CH₂, S, H, Me, 2];
[65: CF₂HCF₂CF₂CF₂CH₂, S, H, Me, 2];
[66: CClF₂CClFCH₂CH₂, S, H, Me, 2];
[67: CBrF₂CF₂CH₂CH₂, S, H, Me, 2];
[68: CF₃CH(Me)CH₂, S, H, Me, 2];
[69: CF₃CH(Me)CH₂CH₂, S, H, Me, 2];
[70: CF₃CF₂CH(Me)CH₂CH₂, S, H, Me, 2];
[71: CF₃CH(Et)CH₂CH₂, S, H, Me, 2];
[72: CF₃CH(Pr)CH₂CH₂, S, H, Me, 2];
[73: CF₃CH(iPr)CH₂CH₂, S, H, Me, 2];
[74: CF₃CH(tBu)CH₂CH₂, S, H, Me, 2];
[75: CF₃CH(OMe)CH₂CH₂, S, H, Me, 2];
[76: CF₃CH(SMe)CH₂CH₂, S, H, Me, 2];
[77: CF₃CH₂CH₂, SO, H, Me, 2];
[78: CF₃CF₂CF₂CH₂, SO, H, Me, 2];
[79: CF₃CF₂CH₂CH₂, SO, H, Me, 2];
[80: CF₃CH₂CH₂CH₂, SO, H, Me, 2];
[81: CF₃CF₂CF₂CH₂CH₂, SO, H, Me, 2];
[82: CF₃CF₂CH₂CH₂CH₂, SO, H, Me, 2];
[83: CF₃CH₂CH₂CH₂CH₂, SO, H, Me, 2];
[84: CF₂HCF₂CF₂CF₂CH₂, SO, H, Me, 2];
[85: CCl₂CClFCH₂CH₂, SO, H, Me, 2];
[86: CBrF₂CF₂CH₂CH₂, SO, H, Me, 2];
[87: CF₃CH(Me)CH₂, SO, H, Me, 2];
[88: CF₃CH(Me)CH₂CH₂, SO, H, Me, 2];
[89: CF₃CF₂CH(Me)CH₂CH₂, SO, H, Me, 2];
[90: CF₃CH(Et)CH₂CH₂, SO, H, Me, 2];
[91: CF₃CH(Pr)CH₂CH₂, SO, H, Me, 2];
[92: CF₃CH(iPr)CH₂CH₂, SO, H, Me, 2];
[93 : CF₃CH(tBu)CH₂CH₂, SO, H, Me, 2];
[94: CF₃CH(OMe)CH₂CH₂, SO, H, Me, 2];
[95: CF₃CH(SMe)CH₂CH₂, SO, H, Me, 2];
[96: CF₃CH₂CH₂, SO2, H, Me, 2];
[97: CF₃CF₂CF₂CH₂, S02, H, Me, 2];
[98: CF₃CF₂CH₂CH₂, SO2, H, Me, 2];
[99: CF₃CH₂CH₂CH₂, S02, H, Me, 2];
[100: CF₃CF₂CF₂CH₂CH₂, SO2, H, Me, 2];
[101: CF₃CF₂CH₂CH₂CH₂, SO2, H, Me, 2];
[102: CF₃CH₂CH₂CH₂CH₂ , SO2, H, Me, 2];
[103: CF₂HCF₂CF₂CF₂CH₂, SO2, H, Me, 2];
[104: CClF₂CClFCH₂CH₂, SO2, H, Me, 2];
[105: CBrF₂CF₂CH₂CH2, SO2, H, Me, 2];
[106: CF₃CH(Me)CH₂, SO2, H, Me, 2];
[107: CF₃CH(Me)CH₂CH₂, SO2, H, Me, 2];
[108: CF₃CF₂CH(Me)CH₂CH₂, SO2, H, Me, 2];
[109: CF₃CH(Et)CH₂CH₂, SO2, H, Me, 2];
[110: CF₃CH(Pr)CH₂CH₂, SO2, H, Me, 2];
[111: CF₃CH(iPr)CH₂CH₂, SO2, H, Me, 2] ;
[112: CF₃CH(tBu)CH₂CH₂, SO2, H, Me, 2] ;
[113: CF₃CH(OMe)CH₂CH₂, SO2, H, Me, 2];
[114 : CF₃CH(SMe)CH₂CH₂ , SO2, H, Me, 2];
[115 : CF₃CH₂CH₂, O, Me, Me, 2];
[116: CF₃CF₂CF₂CH₂, O, Me, Me, 2];
[117: CF₃CF₂CH₂CH₂ , O, Me, Me, 2];
[118: CF₃CH₂CH₂CH₂ , O, Me, Me, 2];
[119 : CF₃CF₂CF₂CH₂CH₂, O, Me, Me, 2];
[120: CF₃CF₂CH₂CH₂CH₂, O, Me, Me, 2];
[121: CF₃CH₂CH₂CH₂CH₂, O, Me, Me, 2];
[122: CF₂HCF2CF₂CF₂CH₂, O, Me, Me, 2];
[123: CClF₂CClFCH₂CH₂, O, Me, Me, 2];
[124: CBrF₂CF₂CH₂CH2, O, Me, Me, 2];
[125: CF₃CH(Me)CH₂, O, Me, Me, 2];
[126: CF₃CH(Me)CH₂CH₂, O, Me, Me, 2];
[127: CF₃CF₂CH(Me)CH₂CH₂, O, Me, Me, 2];
[128: CF₃CH(Et)CH₂CH₂, O, Me, Me, 2];
[129: CF₃CH(Pr)CH₂CH₂, O, Me, Me, 2];
[130: CF₃CH(iPr)CH₂CH₂, O, Me, Me, 2];
[131: CF₃CH(tBu)CH₂CH₂ , O, Me, Me, 2];
[132: CF₃CH(OMe)CH₂CH₂, O, Me, Me, 2];
[133: CF₃CH(SMe)CH₂CH₂, O, Me, Me, 2];
[139: CF₃CH₂CH₂ , O, F, Me, 2];
[135: CF₃CF₂CF₂CH₂, O, F, Me, 2];
[136: CF₃CF₂CH₂CH₂, O, F, Me, 2];
[137: CF₃CH₂CH₂CH₂, O, F, Me, 2];
[138: CF₃CF₂CF₂CH₂CH₂, O, F, Me, 2];
[139: CF₃CF₂CH₂CH₂CH₂, O, F, Me, 2];
[140: CF₃CH₂CH₂CH₂CH₂, O, F, Me, 2];
[141: CF₂HCF₂CF₂CF₂CH₂, O, F, Me, 2];
[142: CClF₂CClFCH₂CH₂, O, F, Me, 2];
[143: CBrF₂CF₂CH₂CH2, O, F, Me, 2];
[144: CF₃CH(Me)CH₂, O, F, Me, 2];
[145: CF₃CH(Me)CH₂CH₂, O, F, Me, 2];
[146: CF₃CF₂CH(Me)CH₂CH₂, O, F, Me, 2];
[147: CF₃CH(Et)CH₂CH₂, O, F, Me, 2];
[148: CF₃CH(Pr)CH₂CH₂, O, F, Me, 2];
[149: CF₃CH(iPr)CH₂CH₂, O, F, Me, 2];
[150: CF₃CH(tBu)CH₂CH₂, O, F, Me, 2];
[151: CF₃CH(OMe)CH₂CH₂, O, F, Me, 2];
[152: CF₃CH(SMe)CH₂CH₂, O, F, Me, 2];
[153: CF₃CH₂CH₂, O, Cl, Me, 2];
[154: CF₃CF₂CF₂CH₂, O, Cl, Me, 2];
[155: CF₃CF₂CH₂CH₂, O, Cl, Me, 2];
[156: CF₃CH₂CH₂CH₂, O, Cl, Me, 2];
[157: CF₃CF₂CF₂CH₂CH₂, O, Cl, Me, 2];
[158: CF₃CF₂CH₂CH₂CH₂, O, Cl, Me, 2];
[159: CF₃CH₂CH₂CH₂CH₂, O, Cl, Me, 2];
[160: CF₂HCF₂CF₂CF₂CH₂, O, Cl, Me, 2];
[161: CClF₂CClFCH₂CH₂, O, Cl, Me, 2];
[162: CBrF₂CF₂CH₂CH₂, O, Cl, Me, 2];
[163: CF₃CH(Me)CH₂, O, Cl, Me, 2];
[164: CF₃CH(Me)CH₂CH₂, O, Cl, Me, 2];
[165: CF₃CF₂CH(Me)CH₂CH₂, O, Cl, Me, 2];
[166: CF₃CH(Et)CH₂CH₂, O, Cl, Me, 2];
[167: CF₃CH(Pr)CH₂CH₂, O, Cl, Me, 2];
[168: CF₃CH(iPr)CH₂CH₂, O, Cl, Me, 2];
[169: CF₃CH(tBu)CH₂CH₂, O, Cl, Me, 2];
[170: CF₃CH(OMe)CH₂CH₂, O, Cl, Me, 2];
[171: CF₃CH(SMe)CH₂CH₂, O, Cl, Me, 2];
[172: CF₃CH₂CH₂, O, Br, Me, 2];
[173: CF₃CF₂CF₂CH₂, O, Br, Me, 2];
[174: CF₃CF₂CH₂CH₂, O, Br, Me, 2];
[175: CF₃CH₂CH₂CH₂, O, Br, Me, 2];
[176: CF₃CF₂CF₂CH₂CH₂, O, Br, Me, 2];
[177: CF₃CF₂CH₂CH₂CH₂, O, Br, Me, 2];
[178: CF₃CH₂CH₂, 0, HC≡CCH₂ , Me, 2];
[179: CF₃CF₂CF₂CH₂, O, HC=CCH₂, Me, 2];
[180: CF₃CF₂CH₂CH₂, O, HC≡CCH₂, Me, 2];
[181: CF₃CH₂CH₂CH₂, O, HC≡CCH₂, Me, 2];
[182: CF₃CF₂CF₂CH₂CH₂, O, HC≡CCH₂, Me, 2];
[183: CF₃CF₂CH₂CH₂CH₂, O, HC≡CCH₂ , Me, 2];
[184: CF₃CH₂CH₂, S, Me, Me, 2];
[185: CF₃CF₂CF₂CH₂, S, Me, Me, 2];
[186: CF₃CF₂CH₂CH₂, S, Me, Me, 2];
[187: CF₃CH₂CH₂CH₂, S, Me, Me, 2];
[188: CF₃CF₂CF₂CH₂CH₂, S, Me, Me, 2];
[189: CF₃CF₂CH₂CH₂CH₂, S, Me, Me, 2];
[190: CF₃CH₂CH₂ , S, F, Me, 2];
[191: CF₃CF₂CF₂CH₂, S, F, Me, 2];
[192: CF₃CF₂CH₂CH₂, S, F, Me, 2];
[193: CF₃CH₂CH₂CH₂, S, F, Me, 2];
[194: CF₃CF₂CF₂CH₂CH₂, S, F, Me, 2];
[195: CF₃CF₂CH₂CH₂CH₂, S, F, Me, 2];
[196: CF₃CH₂CH₂, S, Cl, Me, 2];
[197: CF₃CF₂CF₂CH₂, S, Cl, Me, 2];
[198: CF₃CF₂CH₂CH₂, S; Cl, Me, 2];
[199: CF₃CH₂CH₂CH₂, S, Cl, Me, 2];
[200: CF₃CF₂CF₂CH₂CH₂, S, Cl, Me, 2];
[201: CF₃CF₂CH₂CH₂CH₂, S, Cl, Me, 2];
[202: CF₃CH₂CH₂, S, Br, Me, 2];
[203: CF₃CF₂CF₂CH₂, S, Br, Me, 2];
[204: CF₃CF₂CH₂CH₂, S, Br, Me, 2];
[205: CF₃CH₂CH₂CH₂, S, Br, Me, 2];
[206: CF₃CF₂CF₂CH₂CH₂, S, Br, Me, 2];
[207: CF₃CF₂CH₂CH₂CH₂, S, Br, Me, 2];
[208: CF₃CH₂CH₂, S, HC≡CCH₂ , Me, 2];
[209: CF₃CF₂CF₂CH₂, S, HC≡CCH₂ , Me, 2];
[210: CF₃CF₂CH₂CH₂, S, HC≡CCH₂ , Me, 2];
[211: CF₃CH₂CH₂CH₂, S, HC≡CCH₂ , Me, 2];
[212: CF₃CF₂CF₂CH₂CH₂, S, HC≡CCH₂, Me, 2];
[213: CF₃CF₂CH₂CH₂CH₂, S, HC≡CCH₂ , Me, 2];
[214: CF₃CH₂CH₂, O, H, H, 2];
[215: CF₃CF₂CF₂CH₂ , O, H, H, 2];
[216: CF₃CF₂CH₂CH₂, O, H, H, 2];
[217: CF₃CH₂CH₂CH₂, O, H, H, 2];
[218: CF₃CF₂CF₂CH₂CH₂, O, H, H, 2];
[219: CF₃CF₂CH₂CH₂CH₂, O, H, H, 2];
[220: CF₃CH₂CH₂, O, H, Et, 2];
[221: CF₃CF₂CF₂CH₂, O, H, Et, 2];
[222: CF₃CF₂CH₂CH₂, O, H, Et, 2];
[223: CF₃CH₂CH₂CH₂, O, H, Et, 2];
[224: CF₃CF₂CF₂CH₂CH₂, O, H, Et, 2];
[225: CF₃CF₂CH₂CH₂CH₂, O, H, Et, 2];
[226: CF₃CH₂CH₂, O, H, iPr, 2];
[227: CF₃ CF₂ CF₂ CH₂ , O, H, iPr, 2];
[228: CF₃CF₂CH₂CH₂, O, H, iPr, 2];
[229: CF₃CH₂CH₂CH₂, O, H, iPr, 2];
[230: CF₃CF₂CF₂CH₂CH₂, O, H, iPr, 2];
[231: CF₃CF₂CH₂CH₂CH₂, O, H, iPr, 2];
[232: CF₃CH₂CH₂ , O, H, CF₃, 2];
[233: CF₃ CF₂ CF₂ CH₂ , O, H, CF₃, 2];
[234: CF₃CF₂CH₂CH₂, O, H, CF₃, 2];
[235: CF₃CH₂CH₂CH₂, O, H, CF₃, 2];
[236: CF₃CF₂CF₂CH₂CH₂, O, H, CF₃, 2];
[237: CF₃CF₂CH₂CH₂CH₂, O, H, CF₃, 2];
[238: CF₃CH₂CH₂, O, H, CF₂H, 2];
[239: CF₃CF₂CF₂CH₂, O, H, CF₂H, 2];
[240: CF₃CF₂CH₂CH₂, O, H, CF₂H, 2];
[241: CF₃CH₂CH₂CH₂, O, H, CF₂H, 2];
[242: CF₃CF₂CF₂CH₂CH₂, O, H, CF₂H, 2];
[243: CF₃CF₂CH₂CH₂CH₂, O, H, CF₂H, 2];
[244 : CF₃CH₂CH₂, O, H, CFH₂ , 2];
[245: CF₃CF₂CF₂CH₂, O, H, CFH₂, 2];
[246: CF₃CF₂CH₂CH₂, O, H, CF2, 2];
[247: CF₃CH₂CH₂CH₂, O, H, CFH₂ , 2];
[248: CF₃CF₂CF₂CH₂CH₂, O, H, CFH₂, 2];
[249: CF₃CF₂CH₂CH₂CH₂, O, H, CFH₂ , 2];
[250: CF₃CH₂CH₂, O, H, CF₃CF₂, 2];
[251: CF₃CF₂CF₂CH₂, O, H, CF₃CF₂, 2];
[252: CF₃CF₂CH₂CH₂, O, H, CF₃CF₂, 2];
[253: CF₃CH₂CH₂CH₂, O, H, CF₃CF₂, 2];
[254: CF₃CF₂CF₂CH₂CH₂, O, H, CF₃CF₂, 2];
[255: CF₃CF₂CH₂CH₂CH₂, O, H, CF₃CF₂ , 2];
[256: CF₃CH₂CH₂, O, H, CF₃CH₂, 2] ;
[257: CF₃ CF₂ CF₂ CH₂ , O, H, CF₃CH₂, 2];
[258: CF₃CF₂CH₂CH₂, O, H, CF₃CH₂, 2];
[259: CF₃CH₂CH₂CH₂, O, H, CF₃CH₂, 2];
[260: CF₃CF₂CF₂CH₂CH₂, O, H, CF₃CH₂, 2];
[261: CF₃CF₂CH₂CH₂CH₂, O, H, CF₃CH₂, 2];
[262: CF₃CH₂CH₂, O, H, CF₂Cl, 2];
[263: CF₃CF₂CF₂CH₂, O, H, CF₂Cl, 2];
[264: CF₃CF₂CH₂CH₂, O, H, CF₂Cl, 2];
[265: CF₃CH₂CH₂CH₂, O, H,CF₂Cl, 2];
[266: CF₃CF₂CF₂CH₂CH₂, O, H, CF₂Cl, 2];
[267: CF₃CF₂CH₂CH₂CH₂, O, H, CF₂Cl, 2];
[268: CF₃CH₂CH₂, O, H, CF₂Br, 2];
[269: CF₃CF₂CF₂CH₂, O, H, CF₂Br, 2];
[270: CF₃CF₂CH₂CH₂, O, H, CF₂Br, 2];
[271 : CF₃CH₂CH₂CH₂, O, H, CF₂Br, 2] ;
[272: CF₃CF₂CF₂CH₂CH₂, O, H, CF₂Br, 2];
[273: CF₃CF₂CH₂CH₂CH₂, O, H, CF₂Br, 2];
[274: CF₃CH₂CH₂ , S, H, H, 2];
[275: CF₃CF₂CF₂CH₂, S, H, H, 2];
[276: CF₃CF₂CH₂CH₂, S, H, H, 2];
[277: CF₃CH₂CH₂CH₂, S, H, H, 2];
[278: CF₃CF₂CF₂CH₂CH₂, S, H, H, 2];
[279: CF₃CF₂CH₂CH₂CH₂, S, H, H, 2];
[280: CF₃CH₂CH₂ , S, H, Et, 2];
[281: CF₃CF₂CF₂CH₂, S, H, Et, 2];
[282: CF₃CF₂CH₂CH₂, S, H, Et, 2];
[283: CF₃CH₂CH₂CH₂, S, H, Et, 2];
[284: CF₃CF₂CF₂CH₂CH₂, S, H, Et, 2];
[285: CF₃CF₂CH₂CH₂CH₂, S, H, Et, 2];
[286: CF₃CH₂CH₂, S, H, iPr, 2];
[287: CF₃CF₂CF₂CH₂, S, H, iPr, 2];
[288: CF₃CF₂CH₂CH₂, S, H, iPr, 2];
[289: CF₃CH₂CH₂CH₂, S, H, iPr, 2];
[290: CF₃CF₂CF₂CH₂CH₂, S, H, iPr, 2];
[291: CF₃CF₂CH₂CH₂CH₂, S, H, iPr, 2];
[292: CF₃CH₂CH₂, S, H, CF₃, 2];
[293: CF₃ CF₂ CF₂ CH₂ , S, H, CF₃, 2];
[294: CF₃CF₂CH₂CH₂, S, H, CF₃, 2];
[295: CF₃CH₂CH₂CH₂, S, H, CF₃, 2];
[296: CF₃CF₂CF₂CH₂CH₂, S, H, CF₃, 2];
[297: CF₃CF₂CH₂CH₂CH₂, S, H; CF₃, 2];
[298: CF₃CH₂CH₂, S, H, CF₂H, 2];
[299: CF₃CF_{Z}CF₂CH₂, S, H, CF₂H, 2];
[300: CF₃CF₂CH₂CH₂, S, H, CF₂H, 2];
[301: CF₃CH₂CH₂CH₂, S, H, CF₂H, 2];
[302: CF₃CF₂CF₂CH₂CH₂, S, H, CF₂H, 2];
[303: CF₃CF₂CH₂CH₂CH₂, S, H, CF₂H, 2];
[304: CF₃CH₂CH₂, S, H, CFH₂ , 2];
[305: CF₃CF₂CF₂CH₂, S, H, CFH₂, 2];
[306: CF₃CF₂CH₂CH₂, S, H, CFH₂ , 2];
[307: CF₃CH₂CH₂CH₂, S, H, CFH₂ , 2];
[308: CF₃CF₂CF₂CH₂CH₂, S, H, CFH₂ , 2];
[309: CF₃CF₂CH₂CH₂CH₂, S, H, CFH₂ , 2];
[310: CF₃CH₂CH₂, S, H, CF₃CF₂, 2];
[311: CF₃CF₂CF₂CH₂, S, H, CF₃CF₂, 2];
[312: CF₃CF₂CH₂CH₂, S, H, CF₃CF₂, 2];
[313: CF₃CH₂CH₂CH₂, S, H, CF₃CF₂, 2];
[314: CF₃CF₂CF₂CH₂CH₂, S, H, CF₃CF₂, 2];
[315: CF₃CF₂CH₂CH₂CH₂, S, H, CF₃CF₂, 2];
[316: CF₃CH₂CH₂, S, H, CF₃CH₂, 2];
[317: CF₃CF₂CF₂CH₂, S, H, CF₃CH₂, 2];
[318: CF₃CF₂CH₂CH₂, S, H, CF₃CH₂, 2];
[319: CF₃CH₂CH₂CH₂, S, H, CF₃CH₂, 2];
[320: CF₃CF₂CF₂CH₂CH₂, S, H, CF₃CH₂, 2];
[321: CF₃CF₂CH₂CH₂CH₂, S, H, CF₃CH₂, 2];
[322: CF₃CH₂CH₂, S, H, CF₂Cl, 2];
[323: CF₃CF₂CF₂CH₂, S, H, CF₂Cl, 2];
[324: CF₃CF₂CH₂CH₂, S, H, CF₂Cl, 2];
[325: CF₃CH₂CH₂CH2, S, H, CF₂Cl, 2];
[326: CF₃CF₂CF₂CH₂CH₂, S, H, CF₂Cl, 2];
[327: CF₃CF₂CH₂CH₂CH₂, S, H, CF₂Cl, 2];
[328: CF₃CH₂CH₂, S, H, CF₂Br, 2];
[329: CF₃CF₂CF₂CH₂, S, H, CF₂Br, 2];
[330: CF₃CF₂CH₂CH₂, S, H, CF₂Br, 2];
[331: CF₃CH₂CH₂CH₂, S, H, CF₂Br, 2];
[332: CF₃CF₂CF₂CH₂CH₂, S, H, CF₂Br, 2];
[333: CF₃CF₂CH₂CH₂CH₂, S, H, CF₂Br, 2];
[334: CF₃CH₂CH₂, O, Me, H, 2];
[335: CF₃CF₂CF₂CH₂, Or Me, H, 2];
[336: CF₃CF₂CH₂CH₂, O, Me, H, 2];
[337: CF₃CH₂CH₂CH₂, O, Me, H, 2];
[338: CF₃CF₂CF₂CH₂CH₂, O, Me, H, 2];
[339: CF₃CF₂CH₂CH₂CH₂, O, Me, H, 2];
[340: CF₃CH₂CH₂, O, Me, Et, 2];
[341: CF₃CF₂CF₂CH₂, O, Me, Et, 2];
[342: CF₃CF₂CH₂CH₂, O, Me, Et, 2];
[343: CF₃CH₂CH₂CH₂, O, Me, Et, 2];
[344: CF₃CF₂CF₂CH₂CH₂, O, Me, Et, 2];
[345: CF₃CF₂CH₂CH₂CH₂, O, Me, Et, 2];
[346: CF₃CH₂CH₂, O, Me, iPr, 2];
[347: CF₃CF₂CF₂CH₂, O, Me, iPr, 2];
[348: CF₃CF₂CH₂CH₂, O, Me; iPr, 2];
[349: CF₃CH₂CH₂CH₂, O, Me, iPr, 2];
[350: CF₃CF₂CF₂CH₂CH₂, O, Me, iPr, 2];
[351: CF₃CF₂CH₂CH₂CH₂, O, Me, iPr, 2];
[352: CF₃CH₂CH₂, O, Me, CF₃, 2];
[353: CF₃CF₂CF₂CH₂, O, Me, CF₃, 2];
[354: CF₃CF₂CH₂CH₂, O, Me, CF₃, 2];
[355: CF₃CH₂CH₂CH₂, O, Me, CF₃, 2] ;
[356: CF₃CF₂CF₂CH₂CH₂, O, Me, CF₃ , 2];
[357: CF₃CF₂CH₂CH₂CH₂, O, Me, CF₃, 2];
[358: CF₃CH₂CH₂, O, Me, CF₂H, 2];
[359: CF₃CF₂CF₂CH₂, O, Me, CF₂H, 2];
[360: CF₃CF₂CH₂CH₂, O, Me, CF₂H, 2];
[361: CF₃CH₂CH₂CH₂, O, Me, CF₂H, 2];
[362: CF₃CF₂CF₂CH₂CH₂, O, Me, CF₂H, 2];
[363: CF₃CF₂CH₂CH₂CH₂, O, Me, CF₂H, 2];
[364: CF₃CH₂CH₂, O, Me, CFH₂, 2];
[365: CF₃ CF₂ CF₂ CH₂ , O, Me, CFH₂ , 2];
[366: CF₃CF₂CH₂CH₂, O, Me, CFH₂ , 2];
[367: CF₃CH₂CH₂CH₂, O, Me, CFH₂, 2];
[368: CF₃CF₂CF₂CH₂CH₂, O, Me, CFH₂, 2];
[369: CF₃CF₂CH₂CH₂CH₂, O, Me, CFH₂, 2];
[370: CF₃CH₂CH₂, O, Me, CF₃CF₂, 2];
[371: CF₃CF₂CF₂CH₂, O, Me, CF₃CF₂, 2];
[372: CF₃CF₂CH₂CH₂, O, Me, CF₃CF₂, 2];
[373: CF₃CH₂CH₂CH₂, O, Me, CF₃CF₂, 2];
[374: CF₃CF₂CF₂CH₂CH₂, O, Me, CF₃CF₂, 2];
[375: CF₃CF₂CH₂CH₂CH₂, O, Me, CF₃CF₂, 2];
[376: CF₃CH₂CH₂, O, Me, CF₃CH₂, 2];
[377: CF₃CF₂CF₂CH₂, O, Me, CF₃CH₂, 2];
[378: CF₃CF₂CH₂CH₂, O, Me, CF₃CH₂, 2];
[379: CF₃CH₂CH₂CH₂, O, Me, CF₃CH₂, 2];
[380: CF₃CF₂CF₂CH₂CH₂, O, Me, CF₃CH₂, 2];
[381: CF₃CF₂CH₂CH₂CH₂, O, Me, CF₃CH₂, 2];
[382: CF₃CH₂CH₂, O, Me, CF₂Cl, 2];
[383: CF₃CF₂CF₂CH₂, O, Me, CF₂Cl, 2];
[384: CF₃CF₂CH₂CH₂, O, Me, CF₂Cl, 2];
[385: CF₃CH₂CH₂CH₂, O, Me, CF₂Cl, 2];
[386: CF₃CF₂CF₂CH₂CH₂, O, Me, CF₂Cl, 2];
[387: CF₃CF₂CH₂CH₂CH₂, O, Me, CF₂Cl, 2];
[388: CF₃CH₂CH₂, O, Me, CF₂Br, 2];
[389: CF₃CF₂CF₂CH₂, O, Me, CF₂Br, 2];
[390: CF₃CF₂CH₂CH₂, O, Me, CF₂Br, 2];
[391: CF₃CH₂CH₂CH₂, O, Me, CF₂Br, 2];
[392: CF₃CF₂CF₂CH₂CH₂, O, Me, CF₂Br, 2];
[393: CF₃CF₂CH₂CH₂CH₂, O, Me, CF₂Br, 2];
[394: CF₃CH₂CH₂, O, F, H, 2];
[395: CF₃CF₂CF₂CH₂, O, F, H, 2];
[396: CF₃CF₂CH₂CH₂, O, F, H, 2];
[397: CF₃CH₂CH₂CH₂, O, F, H, 2];
[398: CF₃CF₂CF₂CH₂CH₂, O, F, H, 2];
[399: CF₃CF₂CH₂CH₂CH₂, O, F, H, 2];
[400: CF₃CH₂CH₂, O, F, Et, 2];
[401: CF₃CF₂CF₂CH₂, O, F, Et, 2];
[402: CF₃CF₂CH₂CH₂, O, F, Et, 2];
[403: CF₃CH₂CH₂CH₂, O, F, Et, 2];
[404: CF₃CF₂CF₂CH₂CH₂, O, F, Et, 2];
[405: CF₃CF₂CH₂CH₂CH₂, O, F, Et, 2];
[406: CF₃CH₂CH₂, O, F, iPr, 2];
[407: CF₃CF₂CF₂CH₂, O, F, iPr, 2];
[408: CF₃CF₂CH₂CH₂, O, F, iPr, 2];
[409: CF₃CH₂CH₂CH₂, O, F, iPr, 2] ;
[410: CF₃CF₂CF₂CH₂CH₂, O, F, iPr, 2];
[411: CF₃CF₂CH₂CH₂CH₂, O, F, iPr, 2];
[412: CF₃CH₂CH₂, O, F, CF₃, 2];.
[413: CF₃CF₂CF₂CH₂, O, F, CF₃, 2];
[414: CF₃CF₂CH₂CH₂, O, F, CF₃, 2];
[415: CF₃CH₂CH₂CH₂, O, F, CF₃, 2];
[416: CF₃CF₂CF₂CH₂CH₂, O, F, CF₃, 2];
[417: CF₃CF₂CH₂CH₂CH₂, O, F, CF₃, 2];
[418: CF₃CH₂CH₂, O, F, CF₂H, 2];
[419: CF₃CF₂CF₂CH₂, O, F, CF₂H, 2];
[420: CF₃CF₂CH₂CH₂, O, F, CF₂H, 2] ;
[421: CF₃CH₂CH₂CH₂, O, F, CF₂H, 2];
[422: CF₃CF₂CF₂CH₂CH₂, O, F, CF₂H, 2];
[423: CF₃CF₂CH₂CH₂CH₂, O, F, CF₂H, 2];
[424: CF₃CH₂CH₂, O, F, CFH₂, 2];
[425: CF₃CF₂CF₂CH₂, O, F, CFH₂, 2];
[426: CF₃CF₂CH₂CH₂, O, F, CFH₂, 2];
[427: CF₃CH₂CH₂CH₂, O, F, CFH₂, 2];
[428: CF₃CF₂CF₂CH₂CH₂, O, F, CFH₂, 2];
[429: CF₃CF_{2C}H₂CH₂CH₂, O, F, CFH₂, 2];
[430: CF₃CH₂CH₂, O, F, CF₃CF₂, 2];
[431: CF₃CF₂CF₂CH₂, O, F, CF₃CF₂, 2];
[432: CF₃CF₂CH₂CH₂, O, F, CF₃CF₂, 2];
[433: CF₃CH₂CH₂CH₂, O, F, CF₃CF₂, 2];
[434: CF₃CF₂CF₂CH₂CH₂, O, F, CF₃CF₂, 2];
[435: CF₃CF₂CH₂CH₂CH₂, O, F, CF₃CF₂, 2];
[436: CF₃CH₂CH₂, O, F, CF₃CH₂, 2];
[437: CF₃CF₂CF₂CH₂, O, F, CF₃CH₂, 2];
[438: CF₃CF₂CH₂CH₂, O, F, CF₃CH₂, 2];
[439: CF₃CH₂CH₂CH₂, O, F, CF₃CH₂, 2];
[440: CF₃CF₂CF₂CH₂CH₂, O, F, CF₃CH₂, 2];
[441: CF₃CF₂CH₂CH₂CH₂, O, F, CF₃CH₂, 2];
[442: CF₃CH₂CH₂, O, F, CF₂Cl, 2];
[443: CF₃CF₂CF₂CH₂, O, F, CF₂Cl, 2];
[444: CF₃CF₂CH₂CH₂, O, F, CF₂Cl, 2];
[445: CF₃CH₂CH₂CH₂, O, F, CF₂Cl, 2];
[446: CF₃CF₂CF₂CH₂CH₂, O, F, CF₂Cl, 2];
[447: CF₃CF₂CH₂CH₂CH₂, O, F, CF₂Cl, 2];
[448: CF₃CH₂CH₂, O, F, CF₂Br, 2];
[449: CF₃CF₂CF₂CH₂, O, F, CF₂Br, 2];
[450: CF₃CF₂CH₂CH₂, O, F, CF₂Br, 2];
[451: CF₃CH₂CH₂CH₂, O, F, CF₂Br, 2];
[452: CF₃CF₂CF₂CH₂CH₂, O, F, CF₂Br, 2];
[453: CF₃CF_{2C}H₂CH₂CH₂, O, F, CF₂Br, 2];
[454: CF₃CH₂CH₂, S, Cl, H, 2];
[455: CF₃CF₂CF₂CH₂, S, Cl, H, 2];
[456: CF₃CF₂CH₂CH₂, S, Cl, H, 2];
[457: CF₃CH₂CH₂CH₂, S, Cl, H, 2];
[458: CF₃CF₂CF₂CH₂CH₂, S, Cl, H, 2];
[459: CF₃CF₂CH₂CH₂CH₂, S, Cl, H, 2];
[460: CF₃CH₂CH₂, S, Cl, Et, 2];
[461: CF₃CF₂CF₂CH₂, S, Cl, Et, 2];
[462: CF₃CF₂CH₂CH₂, S, Cl, Et, 2];
[463: CF₃CH₂CH₂CH₂, S, Cl, Et, 2];
[464: CF₃CF₂CF₂CH₂CH₂, S, Cl, Et, 2];
[465: CF₃CF₂CH₂CH₂CH₂, S, Cl, Et, 2];
[466: CF₃CH₂CH₂, S, Cl, iPr, 2] ;
[467: CF₃CF₂CF₂CH₂, S, Cl, iPr, 2];
[468: CF₃CF₂CH₂CH₂, S, Cl, iPr, 2];
[469: CF₃CH₂CH₂CH₂, S, Cl, iPr, 2];
[470: CF₃CF₂CF₂CH₂CH₂, S, Cl, iPr, 2];
[471: CF3CF₂CH₂CH₂CH₂, S, Cl, iPr, 2];
[472: CF₃CH₂CH₂, S, Cl, CF₃, 2];
[473: CF₃CF₂CF₂CH₂, S, Cl, CF₃, 2];
[474: CF₃CF₂CH₂CH₂, S, Cl, CF₃, 2];
[475: CF₃CH₂CH₂CH₂, S, Cl, CF₃, 2];
[476: CF₃CF₂CF₂CH₂CH₂, S, Cl, CF₃, 2];
[477: CF₃CF₂CH₂CH₂CH₂, S, Cl, CF₃, 2];
[478: CF₃CH₂CH₂, S, Cl, CF₂H, 2];
[479: CF₃CF₂CF₂CH₂, S, Cl, CF₂H, 2];
[480: CF₃CF₂CH₂CH₂, S, Cl, CF₂H, 2];
[481: CF₃CH₂CH₂CH₂, S, Cl, CF₂H, 2];
[482: CF₃CF₂CF₂CH₂CH₂, S, Cl, CF₂H, 2];
[483: CF₃CF₂CH₂CH₂CH₂, S, Cl, CF₂H, 2];
[484: CF₃CH₂CH₂, S, Cl, CFH₂, 2];
[485: CF₃CF₂CF₂CH₂, S, Cl, CFH₂, 2];
[486: CF₃CF₂CH₂CH₂, S, Cl, CFH₂, 2];
[487: CF₃CH₂CH₂CH₂, S, Cl, CFH₂, 2];
[488: CF₃CF₂CF₂CH₂CH₂, S, Cl, CFH₂, 2];
[489: CF₃CF₂CH₂CH₂CH₂ , S, Cl, CFH₂ , 2] ;
[490: CF₃CH₂CH₂, S, Cl, CF₃CF₂, 2];
[491: CF₃CF₂CF₂CH₂, S, Cl, CF₃CF₂, 2];
[492: CF₃CF₂CH₂CH₂, S, Cl, CF₃CF₂, 2];

[493: CF₃CH₂CH₂CH₂, S, Cl, CF₃CF₂, 2];
[494: CF₃CF₂CF₂CH₂CH₂, S, Cl, CF₃CF₂, 2];
[495: CF₃CF₂CH₂CH₂CH₂, S, Cl, CF₃CF₂, 2];
[496: CF₃CH₂CH₂, S, Cl, CF₃CH₂, 2];
[497: CF₃CF₂CF₂CH₂, S, Cl, CF₃CH₂, 2];
[498: CF₃CF₂CH₂CH₂, S, Cl, CF₃CH₂, 2];
[499: CF₃CH₂CH₂CH₂, S, Cl, CF₃CH₂, 2];
[500: CF₃CF₂CF₂CH₂CH₂, S, Cl, CF₃CH₂, 2];
[501: CF₃CF₂CH₂CH₂CH₂, S, Cl, CF₃CH₂, 2];
[502: CF₃CH₂CH₂, S, Cl, CF₂Cl, 2];
[503: CF₃CF₂CF₂CH₂, S, Cl, CF₂Cl, 2];
[504: CF₃CF₂CH₂CH₂, S, Cl, CF₂Cl, 2];
[505: CF₃CH₂CH₂CH₂, S, Cl, CF₂Cl, 2];
[506: CF₃CF₂CF₂CH₂CH₂, S, Cl, CF₂Cl, 2];
[507: CF₃CF₂CH₂CH₂CH₂, S, Cl, CF₂Cl, 2];
[508: CF₃CH₂CH₂, S, Cl, CF₂Br, 2];
[509: CF₃CF₂CF₂CH₂, S, Cl, CF₂Br, 2];
[510: CF₃CF₂CH₂CH₂, S, Cl, CF₂Br, 2];
[511: CF₃CH₂CH₂CH₂, S, Cl, CF₂Br, 2];
[512: CF₃CF₂CF₂CH₂CH₂, S, Cl, CF₂Br, 2];
[513: CF₃CF₂CH₂CH₂CH₂, S, Cl, CF₂Br, 2].

A compound represented by the formula (Iβ) : 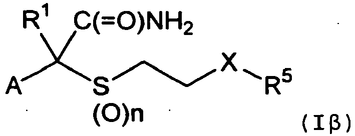 wherein A, X, R¹, R⁵ and n represent any one of combinations shown above.

A compound represented by the formula (Iγ): 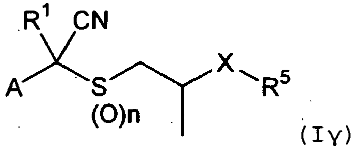 wherein A, X, R¹, R⁵ and n represent any one of combinations shown above.

A compound represented by formula (Iδ): 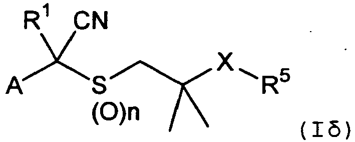 wherein A, X, R¹, R⁵ and n represent any one of combinations shown above.

A compound represented by the formula (Iε): 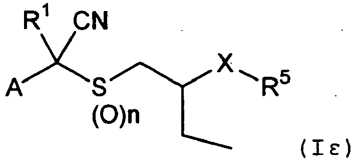 wherein A, X, R¹, R⁵ and n represent any one of combinations shown above.

A compound represented by the formula (Iζ): 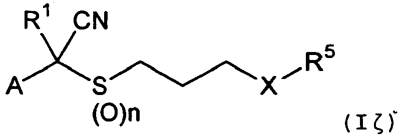 wherein A, X, R¹, R⁵ and n represent any one of combinations shown above.

A compound represented by the formula (Iη): 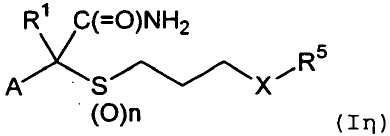 wherein A, X, R¹, R⁵ and n represent any one of combinations shown above.

A compound represented by the formula (Iθ): 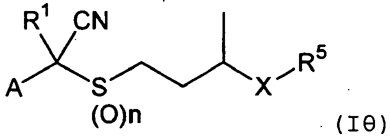 wherein A, X, R¹, R⁵ and n represent any one of combinations shown above.

A compound represented by the formula (I 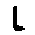 ) : 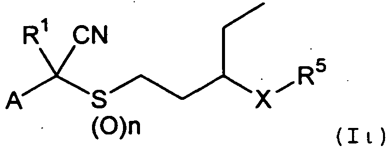 wherein A, X, R¹, R⁵ and n represent any one of combinations shown above.

A compound represented by the formula (Iκ) : 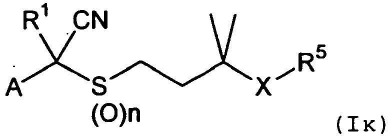 wherein A, X, R¹, R⁵ and n represent any one of combinations shown above.

A compound represented by the formula (Iλ) : 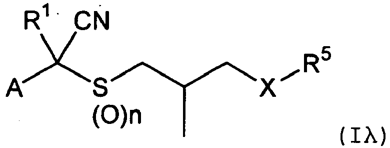 wherein A, X, R¹, R⁵ and n represent any one of combinations shown above.

A compound represented by the formula (Iµ) : 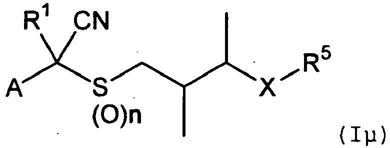 wherein A, X, R¹, R⁵ and n represent any one of combinations shown above.

For example, the present compound (Iζ-47) is a compound having the following structure:

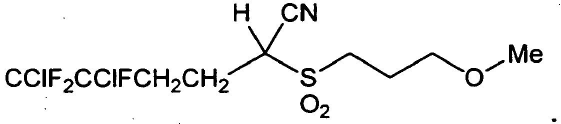

Formulation Examples will be shown below. The term "part(s)" means part(s) by weight.

### Formulation Example 1

Nine parts of any one of the present compounds (1) to (7) is dissolved in 37.5 parts of xylene and 37.5 parts of N,N-dimethylformamide. Thereto 10 parts of polyoxyethylene styryl phenyl ether and 6 parts of calcium dodecylbenzenesulfonate are added and mixed by stirring thoroughly to obtain an emulsifiable concentrate.

### Formulation Example 2

To 40 parts of any one of the present compounds (1) to (7) is added 5 parts of SORPOL 5060 (registered trade name for TOHO Chemical Industry Co., LTD.) and mixed thoroughly. The mixture is mixed with 32 parts of CARPLEX #80 (registered trade name for Shionogi & Co., Ltd., synthetic hydrous silicon oxide fine powder) and 23 parts of 300 mesh diatomaceous earth by using a juice mixer to obtain a wettable powder.

### Formulation Example 3

Three parts of any one of the present compounds (1) to (7), 5 parts of synthetic hydrous silicon oxide fine powder, 5 parts of sodium dodecylbenzenesulfonate, 30 parts of bentonite and 57 parts of clay are mixed by stirring thoroughly. To this mixture an appropriate amount of water is added. The mixture is further stirred, granulated with a granulator, and then air-dried to obtain a granule.

### Formulation Example 4

Four point five parts of any one of the present compounds (1) to (7), 1 part of synthetic hydrous silicon oxide fine powder, 1 part of Dorires B (manufactured by Sankyo) as a flocculant, and.7 parts of clay are mixed thoroughly in a mortar, and then mixed by stirring by using a juice mixer. To the resultant mixture 86.5 parts of cut clay is added and mixed by stirring thoroughly to obtain a dust.

### Formulation Example 5

Ten parts of any one of the present compounds (1) to (7), 35 parts of a mixture (weight ratio of 1:1) of a polyoxyethylene alkylether sulfate ammonium salt and white carbon, and 55 parts of water are mixed and then finely-divided by a wet grinding method to obtain a formulation.

### Formulation Example 6

Zero point five part of any one of the present compounds (1) to (7) is dissolved in 10 parts of dichloromethane. This solution is mixed with 89.5 parts of Isopar M (isoparaffin: registered trade name for Exxon Chemical) to obtain an oil solution.

### Formulation Example 7

Zero point one part of any one of the present compounds (1) to (7) and 49.9 parts of NEO-THIOZOL (Chuo Kasei Co., Ltd.) are placed in an aerosol can. An aerosol valve is fitted to the can. The can is charged with 25 parts of dimethyl ether and 25 parts of LPG. An actuator is fitted to the can to obtain an oily aerosol.

### Formulation Example 8

Zero point six parts of any one of the present compounds (1) to (7), 0.01 part of BHT, 5 parts of xylene, 3.39 parts of a deodorized kerosine and 1 part of an emulsifying agent [Atmos 300 (registered trade name for Atmos Chemical Ltd.)] are mixed to obtain a solution. An aerosol container is charged with the obtained solution and 50 parts of distilled water. A valve part is attached to the container and the container is then charged with 40 parts of a propellant (LPG) through the valve under increased pressure to obtain an aqueous aerosol.

The following Test Examples demonstrate that the compound of the present invention is effective as an active ingredient of an arthropod pest-controlling composition.

### Test Example 1

A formulation of any one of the present compounds (4), (5) and (6) obtained according to Formulation Example 5 was diluted with water to prepare a test solution having 55.6 ppm of the active ingredient.

Separately, 50 g of culture soil, Bonsol No.2 (manufactured by Sumitomo Chemical Co., Ltd.) was put into a polyethylene cup with five holes 5 mm in diameter at the bottom, and 10 to 15 seeds of rice were planted therein. The rice plants were grown until the second foliage leaf was developed, and then treated with 45 ml of the test solution by allowing the plants to absorb the test solution from the bottom of the cup. The rice plants were placed in a greenhouse at 25°C for 6 days and then cut into the same height of 5 cm. Thirty (30) first-instar larvae of *Nilaparvata lugens* were released into the cup, and then the cup was left in a greenhouse at 25°C for 6 days. Then, the number of *Nilaparvata lugens* parasitic on the rice plants was counted.

As a result,' on the plants treated with any one of the present compounds (4), (5) and (6), the number of the parasitic pests was 3 or smaller.

### Test Example 2

A formulation of any one of the present compounds (3), (4) and (5) obtained according to Formulation Example 5 was diluted with water to prepare a test solution having 500 ppm of the active ingredient.

A filter paper having a diameter of 5.5 cm was spread on the bottom of a polyethylene cup having a diameter of 5.5 cm and 0.7 ml of the test solution was added dropwise onto the filter paper. As a bait, 30 mg of sucrose was uniformly placed on the filter paper. Into the polyethylene cup, 10 female imagoes of *Musca domestica* were released and the cup was sealed with a lid. After 24 hours, the number of surviving or dead *Musca domestica* was counted and the death rate of the pest was calculated.

As a result, the treatment with any one of the present compounds (3), (4) and (5) showed a pest death rate of 70% or more.

### Test Example 3

A formulation of any one of the present compounds (3) and (4) obtained according to Formulation Example 5 was diluted with water to prepare a test solution having 500 ppm of the active ingredient.

A filter paper having a diameter of 5.5 cm was spread on the bottom of a polyethylene cup having a diameter of 5.5 cm and 0.7 ml of the test solution was added dropwise onto the filter paper. As a bait, 30 mg of sucrose was uniformly placed on the filter paper. Into the polyethylene cup, 2 male imagoes of *Blattalla germanica* were released and the cup was sealed with a lid. After 6 days, the number of surviving or dead *Blattalla germanica* was counted and the death rate of the pest was calculated.

As a result, the treatment with any one of the present compounds (3) and (4) showed a pest death rate of 100%.

### Test Example 4

A formulation of any one of the present compounds (3) and (4) obtained according to Formulation Example 5 was diluted with water to prepare a test solution having 500 ppm of the active ingredient.

To 100 mL of ion-exchanged water, 0.7 ml of the test solution was added (active ingredient concentration: 3.5 ppm). Into the solution, 20 last-instar larvae of *Culex pipiens pallens* were released. After 1 day, the number of surviving or dead *Culex pipiens pallens* was counted and the death rate of the pest was calculated.

As a result, the treatment with any one of the present compounds (3) and (4) showed a pest death rate of 90% or more.

### Industrial Applicability

The compound of the present invention has a controlling effect on arthropod pests, and is therefore useful as an active ingredient of an arthropod pest-controlling composition.

## Claims

1. A sulfur-containing compound represented by the formula (I): 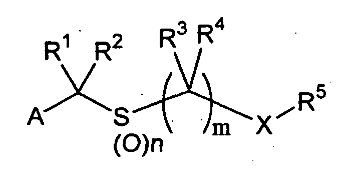 wherein m represents 1, 2 or 3, n represents 0, 1 or 2,
A represents a C2-C10 fluoroalkyl group optionally substituted with a group selected from the group E,
R¹ represents an optionally halogenated C1-C4 chain hydrocarbon group, a halogen atom, or a hydrogen atom,
R² represents an optionally halogenated C1-C4 chain hydrocarbon group, -C(=G¹)R⁶, a cyano group, or a halogen atom,
R³ and R⁴ independently represent an optionally halogenated C1-C4 chain hydrocarbon group, an optionally halogenated phenyl group, a halogen atom, or a hydrogen atom, and when
m represents 2 or 3, two or more R³'s may be the same or different from each other and two or more R⁴'s may be the same or different from each other,
X represents an oxygen atom, a sulfur atom, -SO-, or -SO₂-,
R⁵ represents an optionally halogenated C1-C4 chain hydrocarbon group, -C(=G²)R⁷, a cyano group, or a hydrogen atom,
G¹ and G² independently represent an oxygen atom or a sulfur atom,
R⁶ represents an optionally halogenated C1-C4 alkyl group, a hydroxyl group, an optionally halogenated C1-C4 alkoxy group, an optionally halogenated C3-C6 alkenyloxy group, an optionally halogenated C3-C6 alkynyloxy group, an amino group, an optionally halogenated C1-C4 alkylamino group, an optionally halogenated di(C1-C4 alkyl)amino group, a C2-C5 cyclic amino group, or a hydrogen atom,
R⁷ represents an optionally halogenated C1-C4 chain hydrocarbon group,
The group E consists of -OR⁸, -SR⁸, -SO-R⁸, -SO₂-R⁸, a cyano group, a hydroxyl group, a chlorine atom, and a bromine atom, and
R⁸ represents an optionally halogenated C1-C4 chain hydrocarbon group.

2. The sulfur-containing compound according to claim 1, wherein R² is -C(=G¹)R⁶ or a cyano group.

3. The sulfur-containing compound according to claim 1 or 2, wherein m is 2.

4. The sulfur-containing compound according to claim 1 or 2, wherein m is 2, and R³ and R⁴ are hydrogen atoms.

5. An arthropod pest-controlling composition comprising the sulfur-containing compound according to any one of claims 1 to 4 as an active ingredient.

6. A non-therapeutical method for controlling an arthropod pest, which comprises applying an effective amount of the sulfur-containing compound according to any one of claims 1 to 4 to the arthropod pest or a habitat of the arthropod pest.

## Patentansprüche

1. Eine schwefelhaltige Verbindung, dargestellt durch die Formel (I): 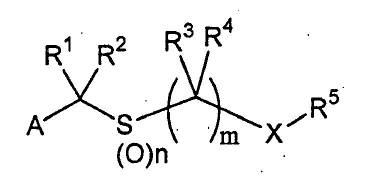 wobei m für 1, 2 oder 3 steht, n für 0, 1 oder 2 steht,
A für einen C2-C10-Fluoralkylrest, gegebenenfalls substituiert mit einem Rest, ausgewählt aus der Gruppe E, steht,
R¹ für einen gegebenenfalls halogenierten C1-C4-Ketten-Kohlenwasserstoffrest, ein Halogenatom oder ein Wasserstoffatom steht,
R² für einen gegebenenfalls halogenierten C1-C4-Ketten-Kohlenwasserstoffrest, -C(=G¹)R⁶, eine Cyanogruppe oder ein Halogenatom steht,
R³ und R⁴ unabhängig für einen gegebenenfalls halogenierten Cl-C4-Ketten-Kohlenwasserstoffrest, eine gegebenenfalls halogenierte Phenylgruppe, ein Halogenatom oder ein Wasserstoffatom stehen und wenn m für 2 oder 3 steht, zwei oder mehrere Reste R³ gleich oder voneinander verschieden sein können und zwei oder mehrere Reste R⁴ gleich oder voneinander verschieden sein können,
X für ein Sauerstoffatom, ein Schwefelatom, -SO- oder -SO₂- steht,
R⁵ für einen gegebenenfalls halogenierten C1-C4-Ketten-Kohlenwasserstoffrest, -C(=G²)R⁷, eine Cyanogruppe oder ein Wasserstoffatom steht,
G¹ und G² unabhängig für ein Sauerstoffatom oder ein Schwefelatom stehen,
R⁶ für einen gegebenenfalls halogenierten C1-C4-Alkylrest, eine Hydroxygruppe, einen gegebenenfalls halogenierten C1-C4-Alkoxyrest, einen gegebenenfalls halogenierten C3-C6-Alkenyloxyrest, einen gegebenenfalls halogenierten C3-C6-Alkinyloxyrest, eine Aminogruppe, einen gegebenenfalls halogenierten C1-C4-Alkylaminorest, einen gegebenenfalls halogenierten Di(C1-C4-Alkyl)aminorest, einen ringförmigen C2-C5-Aminorest oder ein Wasserstoffatom steht,
R⁷ für einen gegebenenfalls halogenierten C1-C4-Ketten-Kohlenwasserstoffrest steht, die Gruppe E aus -OR⁸, -SR⁸, -SO-R⁸, -SO₂-R⁸, einer Cyanogruppe, einer Hydroxygruppe, einem Chloratom und einem Bromatom besteht und
R⁸ für einen gegebenenfalls halogenierten C1-C4-Ketten-Kohlenwasserstoffrest steht.

2. Die schwefelhaltige Verbindung gemäß Anspruch 1, wobei R²-C(=G¹)R⁶ oder eine Cyanogruppe ist.

3. Die schwefelhaltige Verbindung gemäß Anspruch 1 oder 2, wobei m 2 ist.

4. Die schwefelhaltige Verbindung gemäß Anspruch 1 oder 2, wobei m 2 ist und R³ und R⁴ Wasserstoffatome sind.

5. Eine Zusammensetzung zur Bekämpfung eines Arthropodschädlings, umfassend die schwefelhaltige Verbindung gemäß einem der Ansprüche 1 bis 4 als einen Wirkstoff.

6. Ein nicht therapeutisches Verfahren zur Bekämpfung eines Arthropodschädlings, welches das Aufbringen einer wirksamen Menge der schwefelhaltigen Verbindung gemäß einem der Ansprüche 1 bis 4 auf den Arthropodschädling oder einen Lebensraum des Arthropodschädlings umfasst.

## Revendications

1. Composé contenant du soufre représenté par la formule (I) : 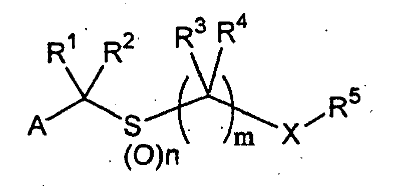 où m représente 1, 2 ou 3, n représente 0, 1 ou 2,
A représente un groupe C2-C10 fluoroalkyle éventuellement substitué avec un groupe choisi dans le groupe E,
R¹ représente un groupe hydrocarboné à chaîne C1-C4 éventuellement halogéné, un atome d'halogène ou un atome d'hydrogène,
R² représente un groupe hydrocarboné à chaîne C1-C4 éventuellement halogéné, -C(=G¹)R⁶, un groupe cyano ou un atome d'halogène,
R³ et R⁴ représentent indépendamment un groupe hydrocarboné à chaîne C1-C4 éventuellement halogéné, un groupe phényle éventuellement halogéné, un atome d'halogène ou un atome d'hydrogène, et quand m représente 2 ou 3, deux ou plusieurs R³ peuvent être identiques ou différents les uns des autres et deux ou plusieurs R⁴ peuvent être identiques ou différents les uns des autres,
X représente un atome d'oxygène, un atome de soufre, -SO- ou -SO₂-,
R⁵ représente un groupe hydrocarboné à chaîne C1-C4 éventuellement halogéné, -C(=G²)R⁷, un groupe cyano ou un atome d'hydrogène,
G¹ et G² représentent indépendamment un atome d'oxygène ou un atome de soufre,
R⁶ représente un groupe C1-C4 alkyle éventuellement halogéné, un groupe hydroxyle, un groupe C1-C4 alcoxy éventuellement halogéné, un groupe C3-C6 alcényloxy éventuellement halogéné, un groupe C3-C6 alcynyloxy éventuellement halogéné, un groupe amino, un groupe C1-C4 alkylamino éventuellement halogéné, un groupe di(C1-C4 alkyl)amino éventuellement un groupe amino C2-C5 cyclique ou un atome d'hydrogène,
R⁷ représente un groupe hydrocarboné à chaîne C1-C4 éventuellement halogéné,
le groupe E consiste en -OR⁸, -SR⁸, -SO-R⁸, -SO₂-R⁸, un groupe cyano, un groupe hydroxyle, un atome de chlore et un atome de brome, et
R⁸ représente un groupe hydrocarboné à chaîne C1-C4 éventuellement halogéné.

2. Composé contenant du soufre selon la revendication 1, où R² est -C(=G¹)R⁶ ou un groupe cyano.

3. Composé contenant du soufre selon la revendication 1 ou 2, où m est 2.

4. Composé contenant du soufre selon la revendication 1 ou 2, où m est 2, et R³ et R⁴ sont des atomes d'hydrogène.

5. Composition de lutte contre un nuisible arthropode comprenant le composé contenant du soufre selon l'une quelconque des revendications 1 à 4 comme ingrédient actif.

6. Procédé non thérapeutique pour la lutte contre un nuisible arthropode, qui comprend l'application d'une quantité efficace du composé contenant du soufre selon l'une quelconque des revendications 1 à 4 au nuisible arthropode ou un habitat du nuisible arthropode.
